# EUROPEAN PATENT APPLICATION

(11) **EP 1 408 119 A1**
(43) Date of publication of application: **14.04.2004**
(21) Application number: 02746113.6
(22) Date of filing: 16.07.2002
(51) Int. Cl.: C12Q 1/68, C12N 1/19, C12N 15/09, G01N 33/15, G01N 33/50, A61K 31/4025, A61P 35/00, A61P 43/00, A61K 45/00, C07D 405/06

(54) **METHOD OF SCREENING ABC PROTEIN PROMOTER/INHIBITOR**

(30) Priority: 18.07.2001 JP 2001218686
(71) Applicant: SUNTORY LIMITED, Osaka-shi, Osaka 530-8203 (JP)
(72) Inventor: ODA, Kohei, Izumi-shi, Osaka 594-1111 (JP); HIRAGA, Kazumi, Kyoto-shi, Kyoto 603-8043 (JP); SHIBANO, Yuji, Toyonaka-shi, Osaka 560-0045 (JP)
(74) Representative: HOFFMANN - EITLE
(86) International application number: PCT/JP2002/007238
(87) International publication number: WO 2003/008644

(57) **Abstract**

The present invention provides a method for efficiently screening a substance which promotes or inhibits an active transfer of ABC protein, characterized in that, (a) ABC protein gene is introduced into a yeast cell having a high drug sensitivity to prepare a transformed yeast cell where ABC protein is expressed in cell membrane site, (b) the transformed yeast cell is incubated in the presence of a test substance and a substance which is subjected to an active transfer outside the cell by ABC protein existing in the cell membrane site and has a cytotoxicity to the yeast cell and (c) degree of the growth of said transformed yeast cell is compared with the case where incubation is carried out in the absence of the test substance.

## Description

### Technical Field

The present invention relates to a method for screening a substance which promotes an active transport function of ABC protein (hereinafter, simply referred to as "a promoting substance for ABC protein") or a substance which inhibits the function (hereinafter, simply referred to as "an inhibiting substance for ABC protein") and to a promoting substance or an inhibiting substance for ABC protein obtained by the screening method.

### Background Art

ABC (ATP binding cassette) protein is a general term for membrane proteins having two well-conserved ATP binding regions (NBF) in a molecule and having 12 to 18 transmembrane domains driven or controlled by ATP. ABC protein is one of the biggest gene families distributed in broad organism species from bacteria to yeasts, plants and mammals and, up to now, not less than 500 ABC protein-related genes have been identified. Further, ABC protein is differentiated to various functions such as transporter, channel and receptor (regulator) and plays an important physiological function in each organism. Particularly in human being, not less than 40 ABC protein genes have been identified now and it has been clarified that abnormality of each gene causes various diseases whereupon importance of the ABC protein as a biodefensive function has been recognized.

Among such ABC proteins, an ABC protein participating in acquisition of multi-drug resistance has been receiving public attention.

An ABC protein participating in acquisition of multi-drug resistance which was firstly identified is MDR1 (also called P-sugar protein) of humans and mice. MDR1 was found during the course of investigation of "acquired multi-drug resistance" which is a problem in cancer therapy where cancer cells acquire resistance to many anti-cancer drugs having different action mechanisms and chemical structures at the same time when an anti-cancer drug is administered. Thus, when genes amplifying in cultured cells which highly acquired anti-cancer resistance are isolated and the genes are expressed in drug-sensitive cells, the cells become resistant to various anti-cancer drugs whereby the genes were called MDR1 which comprises initials of multi-drug resistance (Ueda, K., Cardarelli, C., Gottesman, M. M., and Pastan, I., *Proc*. *Natl. Acad. Sci. USA,* 84, 3004-3008 (1987)). It has been clarified that MDR1 functions as a pump for discharging various fat-soluble drugs from inside of the cells to outside of the cells utilizing the energy of ATP hydrolysis whereupon the cells are made multi-drug resistant.

In addition, MDR1 expresses in capillary endothelium of brain and testis, placenta chorion, membrane at the side of lumen of small intestine and capillary bile duct, renal proximal tubule, hematopoietic stem cells, etc. and, therefore, it is believed to function not only to suppress the invasion of harmful foreign substances into body but also to protect important organs such as brain and fetus from abnormal substances coming into blood (Kazumitsu Ueda, *Tampakushitsu Kakusan Koso*, 46, 588-595 (2001)). Although MDR1 acts as a barrier against harmful substances, it is unable to discharge all fat-soluble harmful substances. Many fat-soluble harmful substances which are unable to be discharged by MDR1 are detoxicated by being conjugated with glutathione or glucuronic acid in cells and discharged outside the cells by MRP1 (multi-drug resistance protein) which is another ABC protein.

The MDR1-analogous gene participating in discharge of foreign substances as such is not expressed in human being only but it has been known that, in yeast for example, there are various kinds of ABC protein genes where substrate specificity is different a little each other (Tokichi Miyagawa and Hidetoshi Takahashi, *Bioscience and Industry,* 58, 397-400 (2000)). It is believed that a function of discharge of foreign substances is probably important in yeast which is unicellular organism and that gene duplication takes place during the course of evolution.

In a budding yeast (yeast belonging to genus *Saccharomyces*), there have been known PDR5 and SNQ2 which are highly analogous to MDR1, STE6 which participates in secretion of yeast-mating factor, YCF1 which participates in discharge of cadmium and YRS1/YOR1 which are organic anion transporters as ABC proteins participating in discharge of foreign substances (Bauer, B. E., et al., *Biochim. Biophys. Acta*, 1461, 217-236 (1999)). Among them, PDR5 is known to discharge various drugs such as cycloheximide, cerulenin, compactin, staurosporine, sulfometuron-methyl, trifluoroperazine and rhodamine outside the cells by means of active transport so as to make yeast resistant to those drugs (Hirata, D. , et al., *Curr*. *Genet*. 26, 285-294 (1994) ) . Although SNQ2 which is a homolog of PDR5 is known to participate in resistance to drugs such as 4-nitroquinoline N-oxide, only two or three compounds are known as the compounds which are able to be discharged by SNQ2 outside the cells (Decottigniest, A., et al., *J*. *Biol*. *Chem*., 270, 1815-18157 (1995)).

As mentioned above, ABC protein is distributed in broad organism species from bacteria to mammals, abnormality of human ABC protein gene is clarified to cause various diseases and importance of ABC protein as a biodefensive function is recognized. Therefore, substances for regulating the active transport function particularly for ABC protein or, in other words, promoting or inhibiting substances for ABC protein have been demanded and a screening method for those substances has been investigated. Particularly with regard to inhibiting substances to ABC protein, they are useful as drugs for suppressing the above-mentioned multi-drug resistance (multi-resistance antagonists) and, therefore, a screening method for the inhibiting substances has been investigated particularly intensively.

As mentioned above, when MDR1 gene is introduced into and expressed in drug-sensitive animal cells, the cells become resistant to various anti-cancer drugs (Ueda, K., et al. , *Proc. Natl. Acad*. *Sci., USA,* 84, 3004-3008 (1987)). A method for screening a drug which inhibits the active transport of MDR1 using the MDR1-expressed drug-resistant cells has been developed. As a result, it has been found that calcium channel blockers such as verapamil inhibit the drug-discharging function by active transport of MDR1 to make the cells sensitive to the drug.

However, the above-mentioned screening method requires much time and labor for the screening of many test substances such as culture supernatant fluid of microbes because of use of animal cells. In addition, since calcium channel blockers per se such as verapamil have a strong cytotoxicity, they are not able to be drugs for overcoming the multi-drug resistance by MDR1.

Therefore, in order to develop simpler and more efficient screening methods for multi-drug resistance antagonists, the present inventors attempted a method where yeast which is an eukaryotic microbe was used as a host instead of animal cell. As a result, it was found that, when human MDR1 gene was expressed in yeast, P-sugar protein which is a product of MDR1 gene was expressed in a membrane fraction of the yeast in the same orientation as the human cells. Then a method for screening a P-sugar protein-inhibiting substance or, in other words, a multi-drug resistance antagonist was developed using a azidopine known to be highly affinitive to P-sugar protein (Japanese Patent Laid-Open No. Hei-2-257873). Thus, the screening method is a method where, in the membrane fraction of yeast in which P-sugar protein is expressed, how much the test substance inhibits the bond of azidopine to P-sugar protein is measured.

However, the above-mentioned method is troublesome in its operation and is insufficient for an efficient screening of many test substances such as culture supernatant liquids of microbes.

In the meanwhile, the present inventors have disclosed substances which inhibit the active transport of PDR5 produced by *Actinomyces* E-420 strain at the General Meeting of Agricultural Chemical Society of Japan in April of 1999 (*Nippon Nogei Kagaku Kaishi*, volume 73, 222 (1999)) and at the General Meeting of Biochemical Society of Japan in September of 1999 (*Seikagaku Kaishi*, volume 71, 812 (1999)).

However, the evaluation of the PDR5-inhibiting substances was carried out by means of changes in sensitivity of the PDR5-expressed yeast to cytotoxic substance or, in other words, changes in growth-inhibiting effect of PDR5-expressed yeast by the cytotoxic substance and, therefore, it is difficult to efficiently evaluate the activity of inhibiting the active transport of PDR5 whereby purification of the inhibiting substance was unable to be conducted.

### Disclosure of the Invention

An object of the present invention is to provide an effective screening method for a promoting substance or an inhibiting substance for ABC protein and also to provide a promoting substance or an inhibiting substances for ABC protein found by the screening method.

In view of a demand for drugs which overcome the multi-drug resistance by amplification and expression of MDR1 gene which is the biggest problem in cancer chemotherapy, a particular object of the present invention is to provide a screening method for a substance which inhibits active transport of MDR1 and has a low cytotoxicity and also to provide a drug which overcomes the multi-drug resistance containing a substance which is found by the screening method.

Another object of the present invention is to provide a drug which contains a promoting substance for ABC protein found by the screening method and which maintains or enhances a biodefensive function so that harmful foreign substances are not invaded into body or organs such as brain and fetus are protected from foreign substances in blood flow.

The present inventors attempted a screening of substances which inhibit the active transfer function of MDR1 using an MDR1-expressing yeast disclosed in the Japanese Patent Laid-Open No. Hei-2-257873. However, since yeasts are inherently resistant to anti-cancer agents, it was difficult to screen the substances which inhibit the active transfer function of MDR1 by means of changes in sensitivity of the MDR1-expressing yeast to anti-cancer agents or changes in growth-inhibiting effect of the MDR1-expressing yeast by anti-cancer agents.

To be more specific, yeasts are inherently and well resistant to anti-cancer agents already and, therefore, it was quite difficult to evaluate an increase in resistance even when MDR1 gene is expressed by the yeast. Thus, with regard to the concentration of an anti-cancer agent for inhibiting the growth of host yeast cells, it was necessary to use the concentration of as high as not less than 1000-fold of the concentration for inhibiting the growth of animal cells and, accordingly, it was substantially impossible from the viewpoint of solubility of the anti-cancer agent to evaluate the resistance to the anti-cancer agent of the transformed yeast where MDR1 gene was expressed. Therefore, since resistance to anti-cancer agent of transformed yeast where MDR1 gene was expressed was unable to be evaluated, it was not possible by means of changes in resistance to the anti-cancer agent to evaluate a test substance which promotes or inhibits the function of discharging the anti-cancer agent as a result of active transport of MDR1 transporter.

In view of the above problems, the present inventors were in an opinion that the biggest problem in an easy and efficient screening method for a promoting substance or an inhibiting substance for ABC protein is that yeast has a high resistance to various kinds of drugs and, as a result of conducting intensive investigations repeatedly, they have created a screening method using a yeast cell having a high drug sensitivity such as SYR1/ERG3 variant as a host and found such a method solves the above-mentioned problem. To be more detail, the above-mentioned problem caused by showing an inherent resistance of yeast cell to an anti-cancer agent has been found to be solved by a method where ABC protein gene is introduced into a yeast cell having a high sensitivity to drug such as SYR1/ERG3 variant, transformed yeast in which the ABC protein is expressed in the cell membrane site is used as an indicator bacterium, a test substance is made to co-exist with a substance which is transported outside the cell by active transport function of the ABC protein such as an anti-cancer agent and the influence of the test substance on the active transport by the ABC protein is evaluated.

To be more specific, there has been created a screening method where (1) SYR1/ERG3 deficient strain which is a synthetic deficient variant of ergosterol which is one of components for constituting yeast cell membrane is a yeast cell having a high drug sensitivity and is used as a host, (2) PDR5 gene which is a multi-drug resistant gene of yeast is introduced as ABC protein gene into the SYR1/ERG3 deficient strain whereupon a transformed yeast is prepared and PDR5 is expressed in cell membrane site of the transformed yeast and, (3) in the co-presence of a test substance and cytotoxic cycloheximide or cerulenin being discharged outside the cell membrane by active transport of PDR5, (4) degree of growth of the indicator bacterium or, in other words, the above-mentioned transformed yeast is measured and is compared with the degree of the growth in the absence of the above test substance. In the screening method, when growth of the indicator bacterium is more suppressed in case growth inhibition of the indicator bacterium by cycloheximide or cerulenin is promoted by the test substance or, in other words, in the case of presence of the test substance as compared with the case of absence of the test substance, then the test substance is an inhibiting substance for ABC protein. On the other hand, when growth of the indicator bacterium more increases in case the growth inhibition of the indicator bacterium by cycloheximide or cerulenin is suppressed by the test substance or, in other words, in the case of presence of the test substance as compared with the case of absence of the test substance, then the test substance is a promoting substance for ABC promoter.

It has then been confirmed that the above-mentioned method is effective as a method for screening a promoting or inhibiting substance for ABC protein using an immunosuppressant FK 506 known as an inhibitor for a PDR5 transporter. Thus, when FK 506 was made to coexist with cycloheximide or cerulenin, growth of the indicator bacterium was suppressed as compared with the case where FK 506 was absent. Thus, discharge of cycloheximide or cerulenin outside the cell by FK 506 was inhibited and growth inhibition of the indicator bacterium by cycloheximide or cerulenin was promoted. In addition, when FK 506 is made to coexist with rhodamine which is a fluorescent dye being known to be discharged outside the cell by PDR5 transporter, then the cellular amount of rhodamine which is a fluorescent dye increased as compared with the case where FK 506 was not coexisting.

Accordingly, when the present screening method is used, a substance having the same action as FK 506 or an inhibitor for ABC protein is able to be easily and efficiently screened. In addition, that is the same for a promoting substance for ABC protein as well.

It is also a characteristic feature of the present invention that, in a screening method for ABC protein inhibitor according to the present invention, when growth inhibition of indicator bacterium by a test substance is evaluated without the coexistence of a substance such as cycloheximide or cerulenin which is discharged outside the cell by active transport of PDR5 in addition to the above-mentioned operations (1) to (4), cytotoxicity of the test substance necessary for evaluation of candidate substances for drug is able to be evaluated at the same time.

The present invention is also able to provide a promoting substance or an inhibiting substance for ABC protein obtained by the above-mentioned screening method. One of the inhibiting substances obtained by the present invention is KPI derived from *Actinomyces* E-420 strain which inhibits the active transport of the yeast PDR5. Moreover, a pharmaceutical composition containing a promoting substance or an inhibiting substance for ABC protein or, to be more specific, a pharmaceutical composition overcoming the multi-drug resistance containing the above-mentioned KPI is a feature of the present invention as well.

Thus, the present invention relates to:
(1) A method for screening a substance which promotes or inhibits an active transfer of ABC protein, characterized in that, (a) ABC protein gene is introduced into a yeast cell having a high drug sensitivity to prepare a transformed yeast cell where ABC protein is expressed in cell membrane site, (b) the transformed yeast cell is incubated in the presence of a test substance and a substance which is subjected to an active transfer outside the cell by ABC protein existing in the cell membrane site and has a cytotoxicity to the yeast cell and (c) degree of the growth of said transformed yeast cell is compared with the case where incubation is carried out in the absence of the test substance;
(2) A method for screening a substance having a low cytotoxicity which promotes or inhibits an active transfer of ABC protein, characterized in that, the substance which promotes or inhibits the active transfer of ABC protein screened by the method mentioned in the above (1) is subjected to measurement of a growth inhibiting action to a yeast cell having a high drug sensitivity where ABC protein is not expressed;
(3) The method for screening mentioned in any of the above (1) and (2), wherein the ABC protein gene is a gene participating in acquisition of multi-drug resistance; and
(4) The method for screening mentioned in the above (3), wherein the gene participating in acquisition of multi-drug resistance is PDR5 gene of yeast.

The present invention further relates to:
(5) The method for screening mentioned in any of the above (1) to (4), wherein the yeast cell having a high drug sensitivity is a variant yeast cell where permeation of drug through cell membrane rises than a wild strain;
(6) The method for screening mentioned in the above (5), wherein the variant yeast cell is a variant having variation to cell membrane lipid synthetic gene;
(7) The method for screening mentioned in the above (6), wherein the variant having variation to the cell membrane lipid synthesis gene is a deficient variant of SYR1/ERG3 gene or gene disruption strain; and
(8) A transformed yeast cell where ABC protein is expressed in a cell membrane site by introduction of ABC protein gene into a yeast cell having a high drug sensitivity.

The present invention still further relates to:
(9) A compound obtained by the method for screening mentioned in any of the above (1) to (7);
(10) A compound represented by a partial structure represented by the following formula (1): 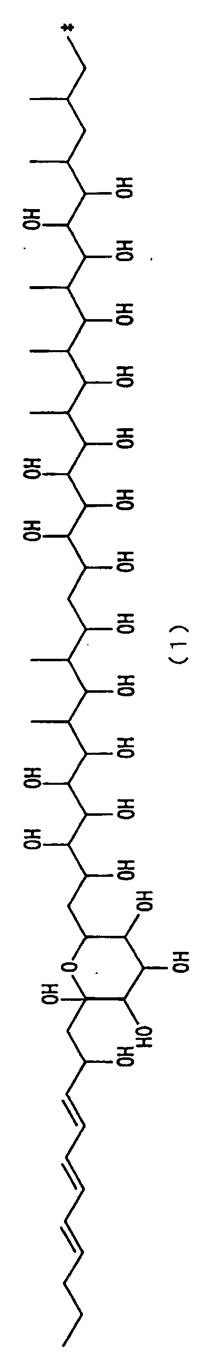 ,having a molecular weight of 1193.7 and having an action of inhibiting a material transport owned by ABC protein or a reduced substance, an ether substance or an ester substance thereof;
   and
(11) The compound mentioned in the above (10) which is obtained from a culture supernatant liquid of *Actinomyces* E-420 strain or a reduced substance, an ether substance or an ester substance thereof.

The present invention furthermore relates to:
(12) An ABC protein-inhibitor which is characterized in containing a compound represented by the formula (2):

   A-B-C (2)

   (in the formula,
   A is a formula (3): 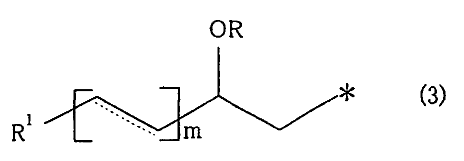 (in the formula, R¹ is hydrogen or a C₁₋₆ lower alkyl; R is hydrogen or a substituent for hydroxy group; m is 3, 4 or 5; and a group in brackets is -CH₂-CH₂- or -CH=CH-.);
   B is a formula (4-1): 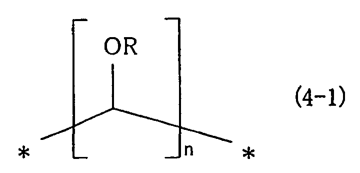 (in the formula, R may be same or different for each constituting unit and is the same as that defined above; and n is 4, 5 or 6) or a formula (4-2): 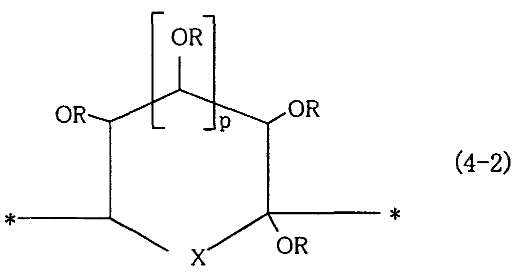 (in the formula, R may be same or different for each constituting unit and is the same as that defined above; p is 0, 1 or 2; and X is O, S or NH);
   C is a formula (5):

      D-E-F-G-Z (5)

      (in the formula, D is the formula (6): 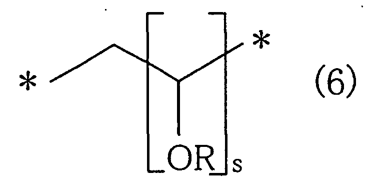 (in the formula, R may be same or different for each constituting unit and is the same as that defined above; and s is 4, 5 or 6);
   E is a formula (7): 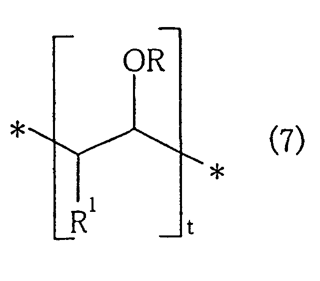 (in the formula, R and R¹ may be same or different for each constituting unit and is the same as that defined above; and t is 2, 3 or 4);
   F is a formula (8): 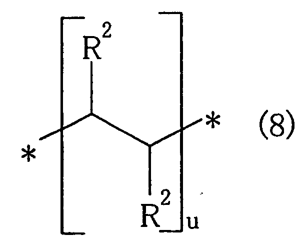 (in the formula, R² may be same or different for each constituting unit or may be same or different in the constituting unit and is OR (R is the same as that defined above) , hydrogen or a C₁₋₆ lower alkyl group; and u is 1 or 2);
   G is a formula (9): 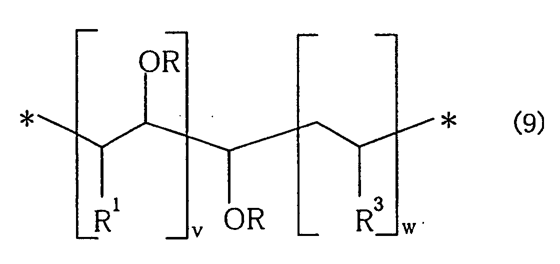 (in the formula, R may be same or different for each constituting unit and is the same as that defined above; R¹ may be same or different for each constituting unit and is the same as that defined above; R³ may be same or different for each constituting unit and is a C₁₋₆ lower alkyl group; v is 3, 4 or 5; and w is 1, 2 or 3); and
   Z is a substituent).

The present invention still furthermore relates to:
(13) The ABC protein-inhibitor mentioned in the above (12), wherein, in the formula (2) mentioned in the above (12), a group represented by the symbol A is the following formula (10): 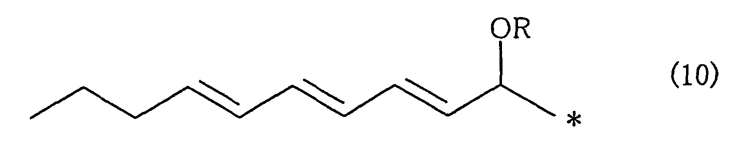 (in the formula, R is hydrogen or a substituent for a hydroxy group);
(14) The ABC protein-inhibitor mentioned in the above (12) or (13), wherein, in the formula (2) mentioned in the above (12), a group represented by the symbol B is the following formula (11): 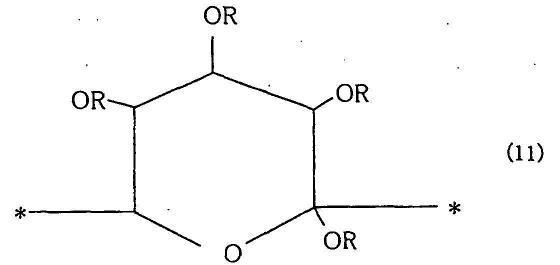 (in the formula, R may be same or different for each constituting unit and is hydrogen or a substituent for a hydroxy group); and
(15) The ABC protein-inhibitor mentioned in any of the above (12) to (14), wherein, in the formula (2) mentioned in the above (12), s is 5, t is 3, u is 1 or 2, v is 4 and w is 2.

The present invention further relates to:
(16) An ABC protein-inhibitor which is characterized in containing a compound represented by the formula (12): 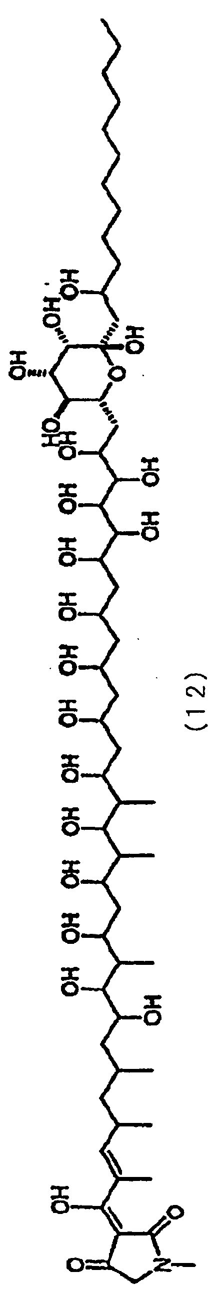 or an ether substance or an ester substance thereof,
   or a compound represented by the formula (13): 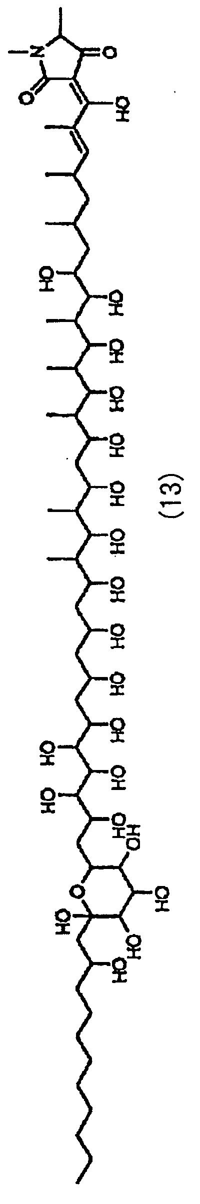 or an ether substance or an ester substance thereof.

The present invention furthermore relates to:
(17) The ABC protein-inhibitor mentioned in any of the above (12) to (16), wherein the ABC protein is an ABC protein participating in acquisition of multi-drug resistance; and
(18) The ABC protein-inhibitor mentioned in the above (17), wherein the ABC protein is PDR5 of yeast.

### Brief Description of the Drawings

Fig. 1 shows the relation between diameter of inhibition ring measured by an agar well diffusion method and logarithm of cycloheximide concentration.
Fig. 2 shows a PDR5 transporter inhibiting activity of a test substance by an agar well diffusion method.
Fig. 3 shows changes in concentration of rhodamine 6G in a cell by a PDR5 transporter. Black dots show KHW3 + pYI-PDR5 while white dots show KHW3 + pYI.
Fig. 4 shows concentrations of rhodamine 6G in a cell as measured by a fluorometer.
Fig. 5 shows a partial structure of KPI by an NMR analysis.
Fig. 6 shows an inhibition activity of KPI to a PDR5 transporter by an agar well diffusion method.
Fig. 7 shows the accumulated amount of rhodamine 123 in a cell to KIP concentration in terms of degree of fluorescence.

### Best Mode for Carrying Out the Invention

With regard to the ABC (ATP binding cassette) protein gene used in the present invention, any gene may be used so far as it has at least two ATP binding regions (NBF) in a molecule and codes for membrane protein having about 6, 12 or 18 times transmembrane domains driven or controlled by ATP. With regard to such an ABC protein gene, MDR1 (Ueda, K., et al., *J*. *Biol*. *Chem*., 262(2), 505-508 (1987)), MRP1 (Cole, S. P., et al., *Science,* 258, 1650-1654 (1992)), SUR1 (Glaser, B, et al., *Nat. Genet*. , 7, 185-188 (1994)), ABC1 (Adams, M. D., et al., *Nature,* 355, 632-634 (1992)), etc. may be exemplified as the gene derived from humans while PDR5 (Goffeau, A. , et al. , *Nature* 387, 98-102 (1997)), SNQ2 (Goffeau, A., et al., *Science* 274, 546 (1996), STE6 (Dujon, B. , et al., *Nature,* 369, 371-378 (1994)), YCF1 (Szczypka, M. S., et al., *J*. *Biol*. *Chem*., 269, 22853-22857 (1994)), YRS1/YOR1 (Goffeau, A., et al., *Science*, 274, 546 (1996), etc. may be exemplified as the gene derived from yeast and PDR5 is used particularly preferably.

In the present invention, it is characteristic to use yeast cells having a high drug sensitivity as a host for introducing the above-mentioned ABC protein gene. Although there is no particular limitation for the yeast cells having a high drug sensitivity, it is preferred to use a variant where permeability of the drug through the yeast cell membrane is enhanced. With regard to such a variant, a variant having a variation in cell membrane lipid synthesis gene is preferred. The cell membrane lipid synthesis variant may be any of known variants. To be more specific, such variants having variation in the cell membrane lipid synthesis gene include, for example, a variant where all or a part of the genes are deficient, a variant where all or a part of the genes are substituted, a variant where a part of the gene are inverted, a variant where a part of the genes are translocated, a variant where a part of the genes are dislocated, a variant where the genes are interrupted by insertion of a gene fragment, etc. With regard to the host used in the present invention, gene disruption strain for cell membrane lipid synthesis gene is advantageously used as well. The gene disruption strain may be prepared in such a manner that gene where a marker gene such as an auxotrophic one is inserted into the cell membrane lipid synthesis gene is prepared and the genetic recombinant DNA prepared as such is introduced into the yeast so as to subject to a homologous recombination with the marker gene on the chromosome.

With regard to the means such as a method for the preparation of gene disruption strain, a method for the transformation of yeast and a method for the preparation of genetic recombinant DNA, they are fully disclosed, for example, in "Molecular Cloning" (2nd ed.) (Cold Spring Harbor Laboratory) (1989).

In the present invention, it is preferred to use a variant deficient in SYR1/ERG3 gene which is a synthetic gene of ergosterol which is one of the constituting components for yeast cell membrane or a gene disruption strain where such a gene is disrupted as the above host. Incidentally, the gene disruption strain of SYR1/ERG3 gene is a gene disruption strain of the SYR1/ERG3 gene prepared by the above means and is a strain where the gene is not translated or synthetic enzyme for ergosterol which is a genetic product of the gene has no normal function or, in other words, a strain where synthesis of ergosterol is not possible. In the present invention, it is particularly preferred to use an SYR1/ERG3 gene deficient strain.

With regard to a method for the introduction of ABC protein gene into yeast cells having a high drug sensitivity in the present invention, it may follow a method which has been known per se such as a method mentioned in "Molecular Cloning" (2nd ed.) (Cold Spring Harbor Laboratory) (1989).

In the transformed yeast prepared as above, it is particularly preferred that ABC protein is expressed at the cell membrane site.

In the screening method according to the present invention, the transformed yeast cells are incubated in the presence of a test substance and a substance (hereinafter, just referred to as "ABC protein transporter") which is transported outside the cells by the ABC protein existing in the cell membrane site and has a cytotoxicity to yeast cells.

The above ABC transporter is discharged outside the cells by active transport of the ABC protein. That is because, as mentioned above, a defensive mechanism of protecting oneself from foreign substances works in the yeast cell. Therefore, in a screening method according to the present invention, the transformed yeast cells are incubated in the co-presence of the above substance and a test substance whereupon influence of the test substance on active transport of ABC protein is able to be evaluated.

There is no particular limitation for the test substance in that case and the substance may be a culture solution of microbes such as *Actinomyces* or a substance synthesized by chemical synthesis. Moreover, it may be protein, a low-molecular substance or a high-molecular substance.

It is preferred that the above ABC protein transporter has cytotoxicity to yeast cells. That is because influence of the test substance on active transport of ABC protein can be easily evaluated by the changes in the growing ability of the transformed yeast. To be more specific, when degree of growth of transformed yeast in the presence of a test substance is more than that in the absence of a test substance, that is because the above ABC protein transporter is discharged from inner area of the cells of the yeast cells and, accordingly, the test substance is able to be evaluated as a substance which promotes the active transport of the ABC protein. On the other hand, when degree of growth of the transformed yeast in the presence of the test substance is less than that in the absence of the test substance, that is because the above ABC protein transporter is accumulated in inner area of the cells of the yeast cells and, accordingly, the test substance is able to be evaluated as a substance which inhibits the active transport of the ABC protein.

In the present invention, there is no particular limitation for the above ABC protein transporter but anything may be used so far as it is transported outside the cells by ABC protein existing in the cell membrane site and has a cytotoxicity to yeast cells. To be specific, fat-soluble substances may be exemplified and, to be more specific, cycloheximide, cerulenin, compactin, staurosporine, sulfometuron-methyl, trifluoroperazine and rhodamine may be exemplified. Those substances are the substances which are able to transport PDR5 of the yeast which was used in Examples.

When degree of growth of transformed yeasts in the presence of a test substance is more than that in the absence of a test substance in the present invention as mentioned above, such a test substance is a substance which promotes an active transport of an ABC protein.

A promoting substance for ABC protein as such is useful as a pharmaceutical composition for maintaining or enhancing the biodefensive mechanism. To be more specific, it is useful as a pharmaceutical composition for maintaining or enhancing the biodefensive mechanism such as (a) a function where toxin coming into capillary endothelium of brain and testis, placenta chorion, membrane at the side of lumen of small intestine and capillary bile duct, renal proximal tubule, hematopoietic stem cells, etc. is discharged or (b) a function where important organs such as brain and fetus are protected from abnormal substances coming into blood flow.

On the other hand, when degree of growth of transformed yeasts in the presence of a test substance is less than that in the absence of a test substance in the present invention as mentioned above, such a test substance is a substance which inhibits an active transport of an ABC protein.

An inhibiting substance for ABC protein as such is useful as a pharmaceutical composition for prevention of acquisition of multi-drug resistance or for suppression of expression of multi-drug resistance. Such a substance is used as a pharmaceutical composition either solely or by way of combination with a drug resulting in resistance by being discharged outside the cells by active transfer of ABC protein. With regard to the above "drug resulting in resistance by being discharged outside the cells by active transfer of ABC protein" , its representative examples are anti-cancer agents such as anthracyclines, vincaalkaloids, etoposide and taxol. When such an anti-cancer agent and the inhibiting substance for ABC protein mentioned above are combined, there is provided a pharmaceutical composition which is also able to be used for the treatment of any cancer expressing the multi-drug resistance. Although there is no particular limitation for the cancer for which the above pharmaceutical composition is useful as a treating agent, it includes, for example, breast cancer, lung cancer, colon cancer, hepatic cancer, renal cancer, pancreatic cancer, prostatic cancer, ovarian cancer, cervical cancer, uterus cancer, bladder cancer, brain tumor, adrenal cancer, multiple myeloma, cancer of ear, nose and throat (including esophagus, laryngeal and pharynx), leukemia, lymphoma, sarcoma and carcinoid tumor.

With regard to a promoting substance or an inhibiting substance for ABC protein obtained by the screening method according to the present invention, there is no particular limitation for its structure, etc.

To be more specific, the following compounds may be listed as inhibitors for ABC protein.

Thus, for example, a compound having a partial structure represented by the following formula (1): 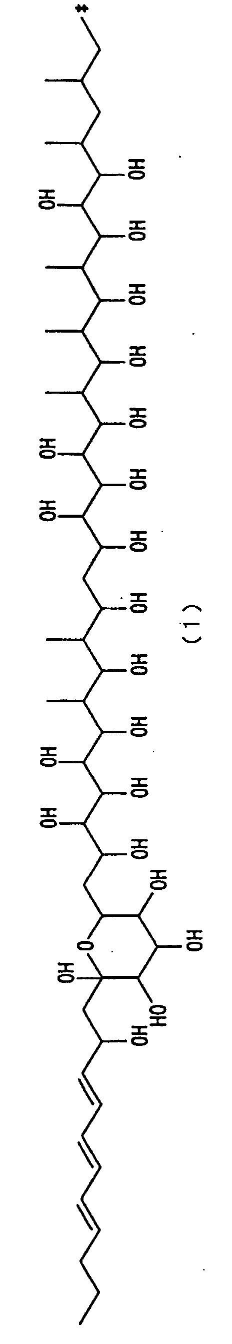 (in the formula, * means CH₂-; it has the same meaning hereinafter as well) having a molecular weight of 1193.7 may be exemplified. Such a compound may be prepared from a culture supernatant liquid of *Actinomyces* E-420 strain or, to be specific, by the method mentioned in the following Examples.

Inhibitors for ABC protein further include reduced substances, ether substances and ester substances of the above compound. Such derivatives can be easily converted to the above compound by reaction means which have been known in the related art.

For example, a reduced substance of the above compound can be prepared by subjecting the compound to the reaction in the presence of a reducing agent. An example of such a method therefor is a method where reducing reaction is carried out at about -30°C to 200°C during the period of from a moment to about seven days in the presence of a reducing agent. Examples of the reducing agent are a metal hydride complex such as sodium borohydride, sodium cyanoborohydride, lithium aluminum hydride, diisobutyl aluminum hydride and sodium triacetoxy borohydride.

A method for the manufacture of the ester substance of the above compound includes a method where solvolysis is carried out using an acid. Thus, for example, the substance can be manufactured by the treatment at the temperature within a range of about 0 to 100°C for about 10 to 24 hours using, for example, formic acid, trifluoroacetic acid, citric acid, hydrochloric acid or sulfuric acid in a solvent such as methylene chloride, anisole, tetrahydrofuran, dioxane, isopropyl acetate, methanol or ethanol or a mixed solvent thereof with water or in the absence of a solvent.

When a solvolysis is carried out using a base, the substance can be manufactured by the treatment with an inorganic base such as sodium hydrogen carbonate, potassium hydrogen carbonate, sodium carbonate or potassium carbonate or with an organic base such as trimethylamine, triethylamine, dimethylaniline or pyridine at the temperature range within about -20 to 80°C for about 10 to 24 hours.

The ether substance of the above compound is able to be manufactured by means of dehydration using an acid catalyst. It is also possible to manufacture by converting the above compound into an alkoxide by a known method *per se* followed by reacting the alkoxide with a haloalkane. Such a reaction has been known as Williamson reaction and, since the reaction means thereof has been well established in the related technical field, that may be followed.

A specific example of the above compound having a partial structure represented by the formula (1) and a molecular weight of 1193.7 is a compound where the group represented by the following group is bonded to the partial structure represented by the formula (1) mentioned above. The compound can be appropriately used as an inhibitor for ABC protein.

Preferred group which is able to bond to the partial structure represented by the formula (1) includes the formula (14-1); 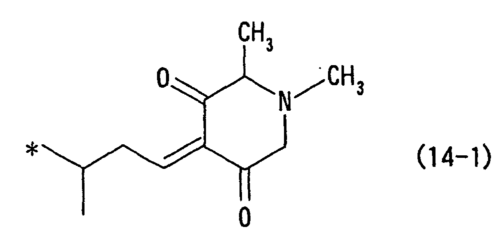 the formula (14-2); 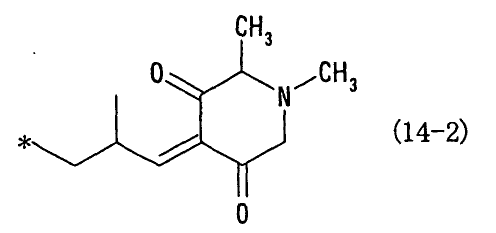 and the formula (14-3);

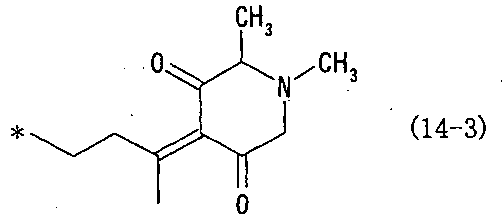

Another specific example of the inhibitor for ABC protein is a compound represented by the formula (2) which is A-B-C.

In the formula (2), a group represented by the symbol A is represented by the formula (3): 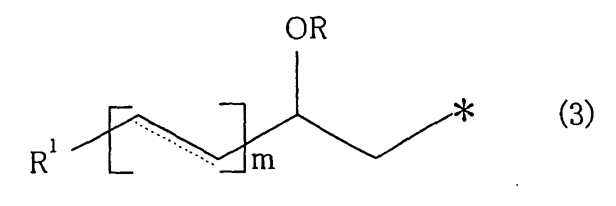 (in the formula, R¹ is hydrogen or a C₁₋₆ lower alkyl group; R is hydrogen or a substituent for hydroxy group; m is 3, 4 or 5; and the moiety in the brackets is -CH₂-CH₂- or -CH=CH-).

It is particularly preferred that the group represented by the symbol A is a group represented by the following formula (10): 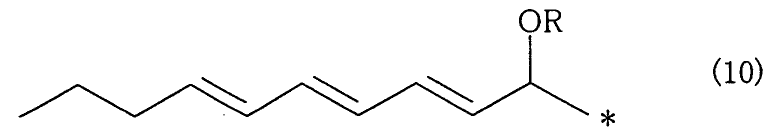 (in the formula, R has the same meaning as defined above).

In the formula (2), a group represented by the symbol B is represented by a formula (4-1): 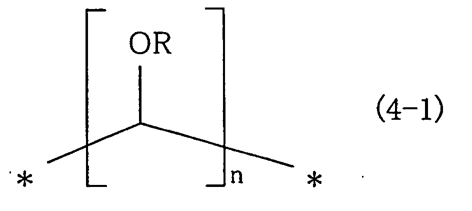 (in the formula, R may be same or different for each constituting unit and is the same as that defined above; and n is 4, 5 or 6) or a formula (4-2): 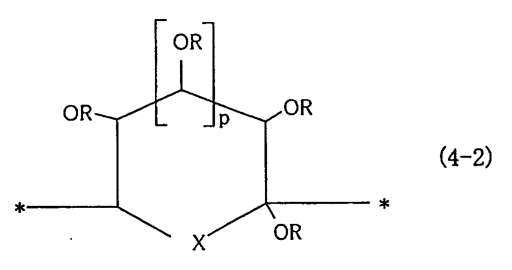 (in the formula, R may be same or different for each constituting unit and is the same as that defined above; p is 0, 1 or 2; and X is O, S or NH).

It is particularly preferred that the group represented by the symbol B is a group represented by the following formula (11): 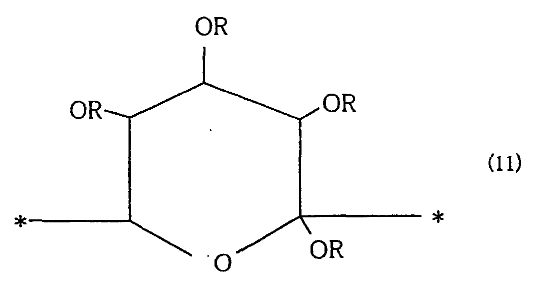 (in the formula, R may be same or different for each substituent and is same as that defined above.)

In the formula (2), a group represented by the symbols C is represented by the formula (5) which is D-E-F-G-Z.

The group represented by the symbol D is represented by the formula (6): 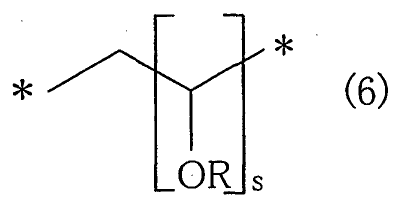 (in the formula, R may be same or different for each constituting unit and is same as that defined above; and s is 4, 5 or 6).

It is particularly preferred when s is 5.

The group represented by the symbol E is represented by the formula (7): 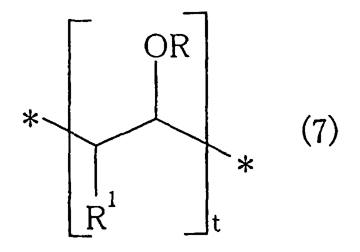 (in the formula, R and R¹ may be same or different for each constituting unit and are same as those defined above; and t is 2, 3 or 4).

It is particularly preferred when t is 3.

The group represented by the symbol F is represented by the formula (8): 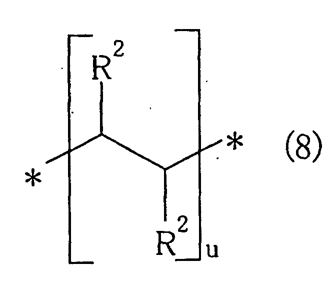 (in the formula, R² may be same or different for each constituting unit and may be same or different in the constituting unit and is OR (R is the same as that defined above), hydrogen or a C₁₋₆ lower alkyl group; and u is 1 or 2).

The group represented by the symbol G is represented by the formula (9): 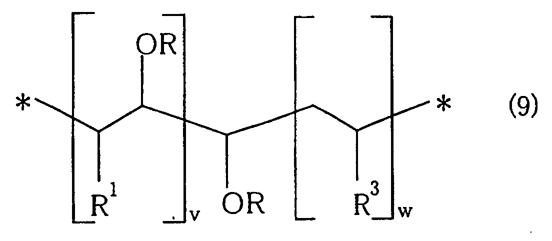 (in the formula, R may be same or different for each constituting unit and is same as that defined above; R¹ may be same or different for each constituting unit and is same as that defined above; R³ may be same or different for each constituting unit and is a C₁₋₆ lower alkyl group; v is 3, 4 or 5; and w is 1, 2 or 3).

It is particularly preferred when v is 4 and w is 2.

The group represented by the symbols Z represents a substituent. Z may be any substituent and it is, for example, a hydrocarbon group, halogen (preferably, fluorine, chlorine or bromine), an alkanoyl group (preferably C₁₋₈), an alkanoyloxy group (preferably C₁₋₈), an alkanoylamino group (preferably C₁₋₈), carboxyl group, an alkoxycarbonyl group (preferably C₂₋₈), a haloalkylcarbonyl group (preferably C₂₋₈), an alkoxy group (preferably C₁₋₈), a haloalkoxy group (preferably C₁₋₈), amino group, an alkylamino group (preferably C₁₋₈), a dialkylamino group (preferably C₁₋₁₆), a cyclic amino group, an alkylaminocarbonyl group (preferably C₂₋₈), carbamoyl group, hydroxy group, nitro group, cyano group, mercapto group, an alkylthio group (preferably C₁₋₈), an alkylsulfonyoxy group (preferably C₁₋₈), an alkylsulfonylamino group (preferably C₁₋₈) or a heterocyclic group.

Such a substituent may be further substituted if desired. There is no particular limitation for type, number and position of the substituent so far as they are within a chemically allowable range.

The above "hydrocarbon group" includes, for example, C₁₋₁₅ alkyl, C₃₋₈ cycloalkyl, C₂₋₁₀ alkenyl, C₃₋₁₀ cycloalkenyl, C₂₋₁₀ alkynyl, C₆₋₁₄ aryl and C₇₋₁₆ aralkyl.

To be more specific, the C₁₋₁₅ alkyl may be either a straight chain or a branched chain and includes, for example, the C₁₋₁₅ alkyl such as methyl, ethyl, propyl, isopropyl, butyl, isobutyl, sec-butyl, tert-butyl, pentyl, isopentyl, neopentyl, hexyl, heptyl, octyl, nonyl and decyl and, preferably, lower (C₁₋₆) alkyl, etc.

The C₃₋₁₀ cycloalkyl includes, for example, cyclopropyl, cyclobutyl, cyclopentyl, cyclohexyl and cycloheptyl.

The C₂₋₁₀ alkenyl may be either a straight chain or a branched chain and includes, for example, vinyl, allyl, crotyl, 2-pentenyl and 3-hexenyl and, preferably, lower (C₂₋₆) alkenyl, etc.

The C₃₋₈ cycloalkenyl includes, for example, 2-cyclopentenyl, 2-cyclohexenyl, 2-cyclopentenylmethyl and 2-cyclohexenylmethyl.

The C₂₋₁₀ alkynyl may be either a straight chain or a branched chain and includes, for example, alkynyl having 2 to 10 carbons such as ethynyl, 1-propynyl, 2-propynyl, 1-butynyl, 2-pentynyl and 3-hexynyl and, preferably, lower (C₂₋₆) alkynyl, etc.

The C₆₋₁₄ aryl includes the C₆₋₁₄ aryl such as phenyl and naphthyl, preferably C₆₋₁₀ aryl or, more preferably, phenyl.

The C₇₋₁₆ aralkyl includes, for example, a phenyl-C₁₋₄ alkyl (such as benzyl and phenethyl).

With regard to the above "alkanoyl group", an alkanoyl group in a straight chain or a branched chain having 1 to 8 carbon(s) is preferred and it includes, for example, formyl group, acetyl group, propionyl group, butyryl group or pivaloyl group.

With regard to the above "alkanoyloxy group", an alkanoyloxy group in a straight chain or a branched chain having 1 to 8 carbon(s) is preferred and it includes, for example, formyloxy group, acetoxy group, propionyloxy group, butyryloxy group or pivaloyoloxy group.

With regard to the above "alkanoylamino group", an alkanoylamino group in a straight chain or a branched chain having 1 to 8 carbon(s) is preferred and it includes, for example, acetylamino group, propionylamino group, butyrylamino group or pivaloyolamino group.

With regard to the above "alkoxycarbonyl group", an alkoxycarbonyl group in a straight chain or a branched chain having 2 to 8 carbons is preferred and it includes, for example, methoxycarbonyl group, ethoxycarbonyl group, propoxycarbonyl group, isopropoxycarbonyl group, butoxycarbonyl group, isobutoxycarbonyl group, tert-butoxycarbonyl group or pentyloxycarbonyl group.

With regard to the above "haloalkylcarbonyl group", a haloalkoxycarbonyl group in a straight chain or a branched chain having 2 to 8 carbons is preferred and it includes, for example, fluoroacetyl group, difluoroacetyl group, trifluoroacetyl group, chloroacetyl group, dichloroacetyl group, trichloroacetyl group, bromoacetyl group, dibromoacetyl group, tribromoacetyl group, 3-chloropropionyl group or 4-chlorobutyryl group. Preferably, fluoroacetyl group, difluoroacetyl group, trifluoroacetyl group, chloroacetyl group, dichloroacetyl group and trichloroacetyl group may be exemplified.

With regard to the above "alkoxy group", an alkoxy group in a straight chain or a branched chain having 1 to 8 carbon(s) is preferred and it includes, for example, methoxy group, ethoxy group, propoxy group, isopropoxy group, butoxy group, tert-butoxy group, pentyloxy group, tert-pentyloxy group or hexyloxy group.

The above "haloalkoxy group" means a group where the above "alkoxy group" is substituted by halogen atom(s) and it includes, for example, fluoromethoxy group, difluoromethoxy group, trifluoromethoxy group, chloromethoxy group, dichloromethoxy group, trichloromethoxy group, bromomethoxy group, dibromomethoxy group, tribromomethoxy group, iodomethoxy group, diiodomethoxy group, triiodomethoxy group, 2-fluoroethoxy group, 2,2-difluoroethoxy group, 2,2,2-trifluoroethoxy group, 2-chloroethoxy group, 2,2-dichloroethoxy group, 2,2,2-trichloroethoxy group, 2-bromoethoxy group, 2,2-dibromoethoxy group, 2,2,2-tribromoethoxy group, 3-chloropropoxy group or 4-chlorobutoxy group.

The above "alkylamino group" means a group where an amino group is substituted by an alkyl group in a straight chain or a branched chain having 1 to 8 carbon(s) and it includes, for example, methylamino group, ethylamino group, propylamino group, isopropylamino group, butylamino group, isobutylamino group, tert-butylamino group, pentylamino group, isopentylamino group, tert-pentylamino group or hexylamino group.

The above "dialkylamino group" means a group where an amino group is substituted by two alkyl groups in a straight chain or a branched chain having 1 to 8 carbon(s) where the type of the amino groups may be same or different. It includes, for example, dimethylamino group, ethyl methylamino group, diethylamino group, methyl propylamino group, ethyl propylamino group, dipropylamino group, diisopropylamino group, dibutylamino group, diisobutylamino group, di-tert-butylamino group, dipentylamino group, diisopentylamino group, di-tert-pentylamino group or dihexylamino group.

The above "cyclic amino group" is a group where an amino group is in a cyclic form. Preferably, it is a four- to eight-membered cyclic amino group and it includes, for example, azetidinyl group, pyrrolidinyl group and piperidino group. It may further have oxygen, sulfur and nitrogen atoms as hetero atoms and may be morpholino group, thiomorpholino group, piperazinyl group, etc. Lower alkyl group or aryl group may be substituted on the nitrogen atom at 4-position of the piperazinyl group.

The above "alkylaminocarbonyl" group is a group where its "alkylamino" moiety is represented by the above "alkylamino group" and it includes, for example, methylaminocarbonyl group, ethylaminocarbonyl group, propylaminocarbonyl group, isopropylaminocarbonyl group, butylaminocarbonyl group, isobutylaminocarbonyl group, tert-butylaminocarbonyl group, pentylaminocarbonyl group, isopentylaminocarbonyl group, tert-pentylaminocarbonyl group or hexylaminocarbonyl group.

With regard to the above "alkylthio group", an alkylthio group in a straight chain or a branched chain having 1 to 8 carbon (s) is preferred and it includes, for example, methylthio group, ethylthio group, propylthio group, isopropylothio group, butylthio group, tert-butylthio group, pentylthio group, tert-pentylthio group or hexylthio group.

With regard to the above "alkylsulfonyloxy group", an alkylsulfonyloxy group in a straight chain or a branched chain having 1 to 8 carbon(s) is preferred and it includes, for example, methylsulfonyloxy group, ethylsulfonyloxy group, propylsulfonyloxy group, isopropylsulfonyloxy group, butylsulfonyloxy group, tert-butylsulfonyloxy group, pentylsulfonyoxy group, tert-pentylsulfonyloxy group or hexylsulfonyloxy group.

The above "alkylsulfonylamino group" is a group where an amino group is substituted by an alkylsulfonyl group in a straight chain or a branched chain having 1 to 8 carbon atom(s) and it includes, for example, methylsulfonylamino group, ethylsulfonylamino group, propylsulfonylamino group, isopropylsulfonylamino group, butylsulfonylamino group, tert-butylsulfonylamino group, pentylsulfonylamino group, tert-pentylsulfonylamino group or hexylsulfonylamino group.

With regard to the heterocyclic group, it includes, for example, a saturated five- to six-membered heterocyclic group having 1 to 2 hetero atom(s) such as tetrahydrofuranyl, tetrahydrothienyl, pyrrolidinyl, pyrazolinyl, piperidyl, piperazinyl, morpholinyl, thiomorpholinyl, tetrahydropyranyl and tetrahydrothiopyranyl. It also includes, for example, a five- to six-membered aromatic heterocyclic ring having 1 to 4 of one or two kind(s) of heteroatom(s) selected from nitrogen atom, sulfur atom and oxygen atom such as furan, thiophene, pyrrole, imidazole, pyrazole, thiazole, oxazole, isothiazole, isoxazole, tetrazole, pyridine, pyrazine, pyrimidine, pyridazine and triazole.

In the compound represented by the above formula (2) A-B-C, there is no particular limitation for the lower alkyl groups represented by R¹, R² and R³ and the above-mentioned substituents are exemplified.

In the compound represented by the above formula (2) A-B-C, there is no particular limitation for the substituent in the hydroxy group represented by R.

To be specific, with regard to a substituent in the hydroxyl group, there is exemplified (1) an optionally substituted hydrocarbon group. With regard to the hydrocarbon group, the above-mentioned substituents are exemplified.

With regard to a substituent in the hydroxy group, there is further exemplified (2) a cycloalkyl group which may contain hetero atom(s). To be more specific, it includes, for example, a saturated five- to six-membered heterocyclic group containing 1 to 2 hetero atom(s) such as tetrahydrofuranyl, tetrahydrothienyl, pyrrolidinyl, pyrazolidinyl, piperidyl, piperazinyl, morpholinyl, thiomorpholinyl, tetrahydropyranyl and tetrahydrothiopyranyl.

With regard to a substituent in the hydroxy group, there is still further exemplified (3) formyl or an optionally substituted acyl. To be more specific, it includes, for example, alkanoyl having 2 to 4 carbons (such as acetyl, propionyl, butyryl and isobutyryl), alkylsulfonyl having 1 to 4 carbon(s) (such as methanesulfonyl and ethanesulfonyl), etc.

Examples of a substituent in (1) an optionally substituted hydrocarbon group or in (3) an optionally substituted acyl group are halogen (such as fluorine, chlorine, bromine and iodine), nitro, cyano, hydroxy group, an optionally substituted thiol group (such as thiol and C₁₋₄ alkylthio), an optionally substituted amino group (such as amino, mono(C₁₋₄ alkyl)amino, di(C₁₋₄ alkyl)amino and five- to six-membered cyclic amino including tetrahydropyrrole, piperazine, piperidine, morpholine, thiomorpholine, pyrrole and imidazole) , an optionally esterified or amidated carboxyl group (such as carboxyl, C₁₋₄ alkoxycarbonyl, carbamoyl, mono(C₁₋₄ alkyl)carbamoyl, di(C₁₋₄ alkyl)carbamoyl, optionally halogenated C₁₋₄ alkyl (such as trifluoromethyl, methyl and ethyl), an optionally halogenated C₁₋₆ alkoxy group (such as methoxy, ethoxy, propoxy, butoxy, trifluoromethyl and trifluoroethoxy), formyl, C₂₋₄ alkanoyl (such as acetyl and propionyl), C₁₋₄ alkylsulfonyl (such as methanesulfonyl and ethanesulfonyl), an optionally substituted five- to six-membered aromatic hetero ring (such as a five- to six-membered aromatic hetero ring containing 1 to 4 of one or two kind(s) of hetero atom(s) selected from nitrogen atom, sulfur atom and oxygen atom including furan, thiophene, pyrrole, imidazole, pyrazole, thiazole, oxazole, isothiazole, isoxazole, tetrazole, pyridine, pyrazine, pyrimidine, pyridazine and triazole; incidentally, a substituent which the hetero ring may have includes, for example, halogen (e.g., fluorine, chlorine, bromine and iodine), nitro, cyano, hydroxy group, thiol group, amino group, carboxyl group, optionally halogenated C₁₋₄ alkyl (e.g., trifluoromethyl, methyl and ethyl), optionally halogenated C₁₋₄ alkoxy (e.g., methoxy, ethoxy, propoxy, butoxy, trifluoromethoxy and trifluoroethoxy), formyl, C₂₋₄ alkanoyl (e.g., acetyl and propionyl), C₁₋₄ alkylsulfonyl (e.g., methanesulfonyl and ethanesulfonyl), etc.) and the like.

The compound represented by the formula (2) can be easily manufactured by a method which has been publicly known in the related art from the above compound being able to be easily isolated from a culture supernatant liquid of *Actinomyces* and having a partial structure of the formula (1).

The inhibiting substance for ABC protein according to the present invention may be a pharmacologically acceptable salt of the compound represented by the formula (2). The "pharmacologically acceptable salt" includes, for example, salt with an inorganic base, salt with an organic base, salt with an inorganic acid, salt with an organic acid and salt with a basic or acidic amino acid.

Appropriate examples of the salt with an inorganic base are alkali metal salts such as sodium salt and potassium salt; alkaline earth metal salts such as calcium salt and magnesium salt; aluminum salt; ammonium salt; etc.

Appropriate examples of the salt with an organic base are salts with trimethylamine, triethylamine, pyridine, picoline, ethanolamine, diethanolamine, triethanolamine, dicyclohexylamine and N,N'-dibenzylethylenediamine.

Appropriate examples of the salt with a basic amino acid are salts with arginine, lysine, ornithine, etc. while appropriate examples of the salt with an acidic amino acid are salts with aspartic acid, glutamic acid, etc.

Appropriate examples of the salt with an inorganic acid are salts with hydrochloric acid, hydrobromic acid, nitric acid, sulfuric acid, phosphoric acid, etc.

Appropriate examples of the salt with an organic acid are salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.

Other specific compounds of the inhibiting substance for ABC protein according to the present invention are the compound represented by the following formula (12) 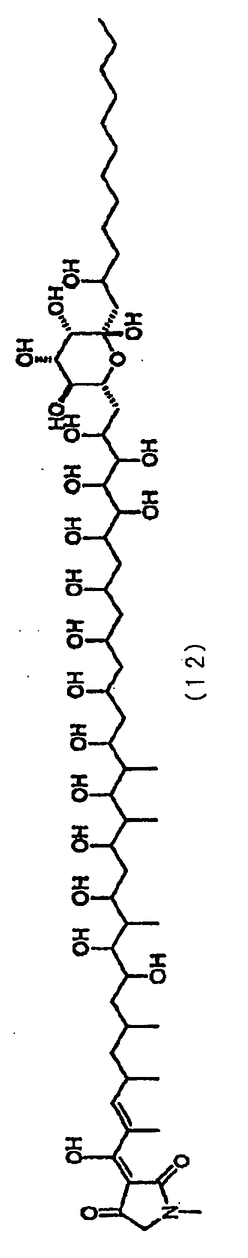 and an ether substance and an ester substance thereof.

Still other specific compounds of the inhibiting substance for ABC protein according to the present invention are the compound represented by the following formula (13) 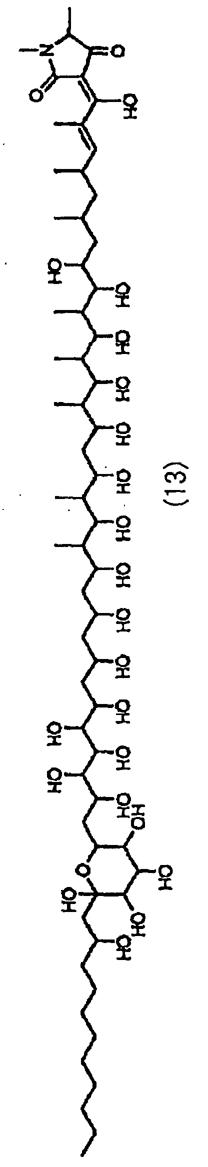 and an ether substance and an ester substance thereof.

Incidentally, the ether substance or the ester substance of the compound represented by the above formula (12) or (13) can be easily manufactured by a known method which was mentioned already.

The present invention provides a pharmaceutical composition containing the above promoting substance or inhibiting substance for ABC protein.

Such a pharmaceutical composition is able to be used to mammals (such as human being, mouse, rat, rabbit, dog, cat, cattle, horse, pig and monkey) as the above promoting substance or inhibiting substance for protein as it is or as a mixture with a pharmaceutically acceptable carrier or the like known *per se*.

Dose depends upon various factors such as type of the diseases, degree of seriousness of the state, age, body weight and sexuality of the patient, type of diagnostic or treating substances used, etc. Skilful clinicians are able to easily decide or formulate the effective dose of a compound or a pharmaceutical composition necessary for diagnosis or treatment of the patient. To be simple, persons skilled in the art use relatively small dose in the initial stage and then increase the dose until the maximum response is achieved.

The pharmaceutical composition according to the present invention may be in a known form such as a solid preparation (e.g., tablets, capsules, granules and powder preparation), a liquid preparation (e.g., syrup and injection), etc. Moreover, the pharmaceutical composition according to the present invention may be administered either orally or parenterally.

With regard to the above "pharmaceutically acceptable carrier", various organic or inorganic carrier substances which have been commonly used as materials for pharmaceutical preparations may be used and it includes fillers, lubricants, binders and disintegrating agents for solid preparations; solvents, solubilizer, suspending agents, isotonizing agents, buffers and soothing agents for liquid preparations; etc.

It is also possible to use additives for pharmaceutical preparations such as antiseptics, antioxidants, coloring agents and sweeteners if necessary.

Appropriate examples of the filler are lactose, sucrose, D-mannitol, starch, crystalline cellulose and light anhydrous silicic acid. Appropriate examples of the lubricant are magnesium stearate, calcium stearate, talc and colloidal silica.

Appropriate examples of the binder are crystalline cellulose, sucrose, D-mannitol, dextrin, hydroxypropyl cellulose, hydroxypropyl methyl cellulose and polyvinylpyrrolidone.

Appropriate examples of the disintegrating agent are starch, carboxymethyl cellulose, carboxymethyl cellulose calcium, croscarmellose calcium and carboxymethyl starch sodium.

Appropriate examples of the solvent are water for injection, alcohol, propylene glycol, macrogol, sesame oil and corn oil.

Appropriate examples of the solubilizer are polyethylene glycol, propylene glycol, D-mannitol, benzyl benzoate, ethanol, trisaminomethane, cholesterol, triethanolamine, sodium carbonate and sodium citrate.

Appropriate examples of the suspending agent are surface-active agent such as stearyl triethanolamine, sodium lauryl sulfate, laurylaminopropionic acid, lecithin, benzalkonium chloride, benzethonium chloride and glycerol monostearate; hydrophilic polymer such as polyvinyl alcohol, polyvinylpyrrolidone, carboxymethyl cellulose sodium, methyl cellulose, hydroxymethyl cellulose, hydroxyethyl cellulose and hydroxypropyl cellulose; etc.

Appropriate examples of the isotonizing agent are sodium chloride, glycerol and D-mannitol.

Appropriate examples of the buffer are buffer solutions such as phosphate, acetate, carbonate and citrate.

Appropriate examples of the soothing agent are benzyl alcohol, etc.

Appropriate examples of the antiseptic agent are p-hydroxybenzoates, chlorobutanol, benzyl alcohol, phenethyl alcohol, dehydroacetic acid and sorbic acid.

Appropriate examples of the antioxidant are sulfates and ascorbic acid.

### Examples

### [Example 1] Construction of screening system for an inhibiting substance for PDR5 transporter

Due to its low permeability of cell membrane for drugs, yeast of genus *Saccharomyces* has a high resistance to drugs and it was not possible to use the yeast of genus *Saccharomyces* for screening of PDR5 transporter inhibitors by way of changes to sensitivity to drugs.

On the other hand, it has been known that sensitivity of an ergosterol synthesis variant (Δsyr1/erg3) of yeast of genus *Saccharomyces* to various drugs becomes high (Hemmi, K., et al., *Biosci*. *Biotech*. *Biochem*., 59, 482-486 (1995)) and, with a presumption that deficiency of ergosterol of cell membrane increases the permeability of the cell membrane, an ergosterol synthesis variant (Δsyr1/erg3) was used as an indicator bacterium in the screening of a PDR5 transporter-inhibiting substance.

A double gene disruption strain KHW2 deficient in PDR5 (Δsyr1/erg3::HIS Δpdr5::LEU2), a double gene disruption strain KHW4 deficient in SNQ2 which is another drug-discharging ABC transporter of yeast (Δpdr5::LEU2 Δsnq2::HIS3) and a triple gene disruption strain KHW2 deficient in PDR5 and SNQ2 (Δsyr1/erg3::HIS3 Δpdr5::LEU2 Δsnq2::HIS3) were prepared from an SYR1/ERG3 gene disruption strain KHW1 of *Saccharomyces cerevisiae* W303-1A (wild type) (Δsyr1/erg3::HIS3). Preparation of multiple gene disruption strains was carried out in such a manner that a host strain was transformed by a plasmid for disruption of gene where LEU2 gene or LEU3 gene was a marker and the disrupted strain where each drug-resistant gene was disrupted by insertion of LEU2 or HIS3 gene was selected using recovery of requirement of leucine or histidine as an index. Each of the multiple gene disruption strains was confirmed by an increase in sensitivity to cycloheximide and cerulenin while gene disruption of PDR5 and SNQ2 was confirmed by a southern hybridization.

Table 1 shows the result of drug sensitivity as measured in terms of the minimum inhibition concentration (MIC) to cycloheximide and cerulenin of yeast where pYI-PDR5 or pYI in which PDR5 gene was inserted to a highly copied plasmid pYI (Hirata, D., et al., *Curr. Genet.*, 26, 285-294 (1994)) using each of the above drug-resistant gene disruption strain and wild strain as a host. Measurement of the MIC was carried out in such a manner that a suspension of each strain in sterilized water (1 × 10⁷ cells/ml) was spotted on an SD-uracil agar medium (Adams, A., et al., Methods in Yeast Genetics, Cold Spring Harbor Laboratory Press, 147-149 (1997)) containing a drug of various concentrations so as to make it about 4 × 10⁴ cells per spot, incubation was carried out at 32°C for two days and the minimum concentration of the drug where no growth of each yeast strain was noted was determined. Result of the measurement is shown in Table 1.

**Table 1**

| Yeast Strain | MIC (µg/ml) | |
|---|---|---|
| | Cycloheximide | Cerulenin |
| W303-1A + pYI | 0.16 | 0.8 |
| KHW1 + pYI | 0.04 | 0.4 |
| KHW3 + pYI | 0.01 | 0.2 |
| KHW3 + pYI-PDR5 | 0.16 | 0.8 |
| KHW4 + pYI | 0.02 | 0.4 |
| KHW4 + pYI-PDR5 | >0.32 | 0.6 |

Sensitivity of the SYR1 gene disruption strain (KHW1 + pYI) to cycloheximide and cerulenin increased to an extent of 4-fold and 2-fold, respectively as compared with the wild strain (W303-1A + pYI). The MIC values of the triple gene disruption strain KHW3 to cycloheximide and cerulenin were 0.01 µg/ml and 0.2 µg/ml, respectively while those of the wild strain W303-1A to cycloheximide and cerulenin were 0.16 µg/ml and 0.8 µg/ml, respectively. Accordingly, the result is that sensitivity of the triple gene disruption strain KHW3 to cycloheximide and cerulenin increased to an extent of 16-fold and 4-fold, respectively as compared with the wild strain.

Then a method for screening of a substance which inhibits the discharge of cycloheximide and cerulenin by a PDR5 transporter according to a growth inhibition assay by means of an agar well diffusion method was constructed.

Yeast cells grown on an SD-uracil medium were suspended in a soft agar dissolved in 4 ml at 50°C so as to make 1 × 10⁵ cells/ml, mixed after addition of a drug such as cycloheximide or cerulenin if necessary and layered on an SD-uracil medium containing the drug of the same concentration. After the soft agar was solidified, a pore of 8 mm diameter was formed on a part of the soft agar and 50 µl of the test substance were poured thereinto. The plate was incubated at 32°C for 48 hours and concentration of growth inhibition ring around the well was measured whereupon activity of the test substance for inhibiting the discharge of cycloheximide and cerulenin by the PDR transporter was evaluated.

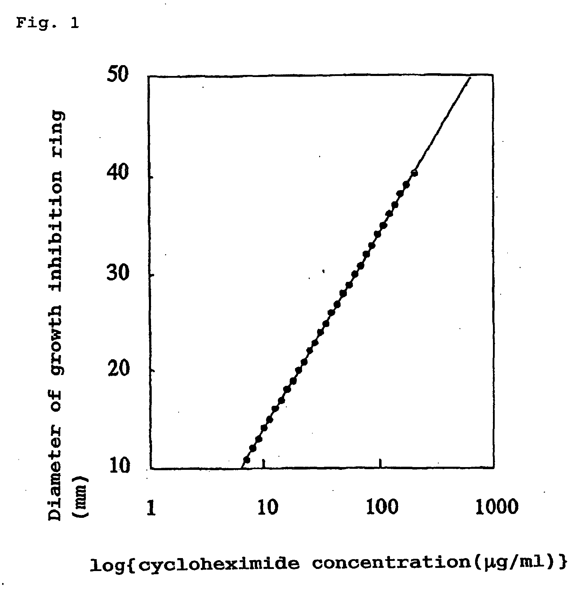

Unit for the growth inhibition activity was defined as follows. Thus, when a triple gene disruption strain KHW3 (Δsyr1/erg3::HIS3 Δpdr5::LEU2 Δsnq2::HIS3) was used and diameter of the inhibition ring measured by the above agar well diffusion method was plotted to logarithm of cycloheximide concentration where cycloheximide of 8-200 µg/ml was used as a standard substance, it was noted there was a linear relation as shown in Fig. 1. Growth inhibition activity showing an inhibition ring of 20 mm to KHW3 containing pYI was defined as 20 inhibition units per ml (IU/ml) and, using the above-mentioned standard curve of cycloheximide, inhibition units of test substances were calculated.

Four kinds of assay plates were prepared in order to know appropriate concentrations of cycloheximide and cerulenin in conducting the screening of a PDR5 transporter-inhibiting substance by the above agar well diffusion method. (1) An assay plate where a triple gene disruption strain KHW3 (Δsyr1/erg3::HIS3 Δpdr5::LEU2 Δsnq2::HIS3) containing pYI was spread on an SD-uracil agar medium in order to check cytotoxicity of the test substance; (2) an assay plate where a triple gene disruption strain KHW3 containing pYI-PDR5 was spread on an SD-uracil agar medium containing no drug in order to highly express the PDR5 gene; (3) an assay plate where the same transformed yeast as in (2) was spread on an SD-uracil agar medium containing cycloheximide; and (4) an assay plate where the same transformed yeast as in (2) was spread on an SD-uracil agar medium containing cerulenin.

As shown in Table 1, the result where MICs of a triple gene disruption strain KHW3 containing pYI-PDR5 and KHW3 containing pYI vector were measured using the four kinds of assay plates, concentrations of cycloheximide and cerulenin which did not inhibit the growth of the triple gene disruption strain KHW3 containing pYI-PDR5 and caused a growth inhibition of KHW3 containing a vector pYI were defined as 0.04 µg/ml and 0.4 µg/ml, respectively and screening of a PDR5 transporter-inhibiting substance at those concentrations was carried out.

A test substance which inhibited the function of a PDR5 transporter but which itself had no cytotoxicity did not form an inhibition ring in the plates of (1) and (2) and, in the plates of (3) and (4), function of a PDR5 transporter which discharged the cellular cycloheximide and cerulenin outside the cells was inhibited whereupon inhibition ring was formed. In the case of an inhibiting substance for a PDR5 transporter although the substance per se showed cytotoxicity, inhibition rings were formed even in the plates of (1) and (2) and, in the plates of (3) and (4), larger inhibition rings than in the plates of (1) and (2) were formed. In that case, when the PDR5 transporter-inhibiting substance *per se* was discharged outsides the cell by the PDR5 transporter, no inhibition ring was formed in the plate of (2) although smaller inhibition rings were formed than in the cases of the plates of (1), (3) and (4). Accordingly, this method is shown to bean efficient screening method for a PDR5 transporter-inhibiting substance having no cytotoxicity.

### [Example 2] Test of a screening method for a PDR5 transporter-inhibiting substance by FK 506

Since FK 506 which is an immunosuppressant inhibits the transport of dexamethasone and azole by a PDR5 transporter, it is presumed to be a PDR5 transporter-inhibiting substance (Egner, R., et a., *Mol. Biol*. *Cel*., 9, 523-543 (1998)) and a growth inhibiting effect of FK 506 or, in other words, a PDR5 transporter-inhibiting activity in the above screening method was investigated using the FK 506 as a model compound whereupon a screening method for a PDR5 transport-inhibiting substance in the above agar well diffusion method was tested.

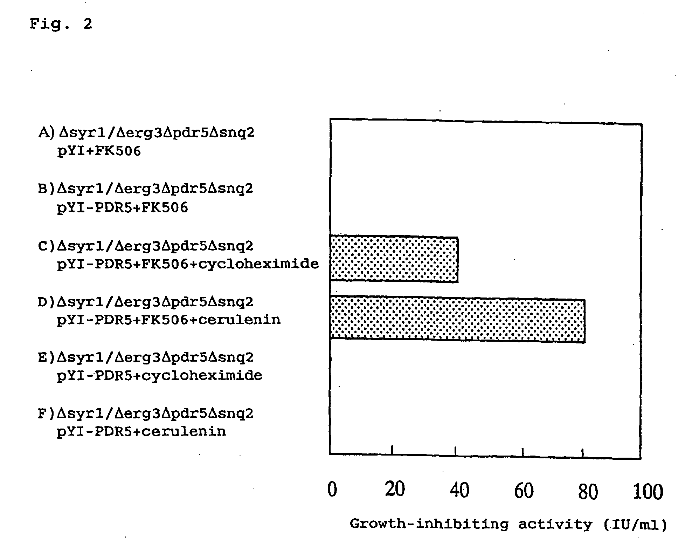

A triple gene disruption strain KHW3 containing pYI vector (KHW + pYI) and KHW3 containing a plasmid pYI-PDR5 highly expressing PDR5 (KHW3 + pYI-PDR5) were used as indicator bacteria, and cycloheximide and cerulenin were added to an SD-uracil medium to make them 0.04 µg/ml and 0.4 µg/ml, respectively. An FK 506 solution (12.5 µg/ml) (50 µl) was added to a well and incubated at 32°C for 48 hours. As shown in Fig. 2, FK 506 did not inhibit the growth of KHW3 + pYI and also did not inhibit the growth of KHW3 + pYI-PDR5 although the growth of KHW3 + pYI-PDR5 was inhibited when cycloheximide was made to coexist with FK 506 or when cerulenin was made to coexist with FK 506. Growth of KHW3 + pYI-PDR5 was not inhibited by a sole cycloheximide or cerulenin. The result shows that FK 506 inhibited the function of a PDR5 transporter or the discharge of the drug outside the cells and that, as a result, cycloheximide or cerulenin was accumulated in the yeast cells and growth of the yeast was inhibited.

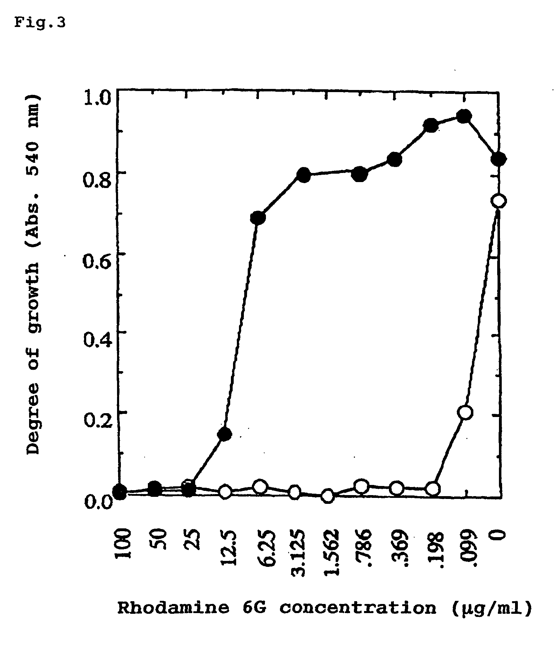

In order to confirm that FK 506 inhibits the drug-discharging function of a PDR5 transporter, effect of FK 506 on the discharge of rhodamine 6G outside the cells was investigated. It has been known that rhodamine 6G is discharged outside the cell by a PDR5 transporter (Kolaczkowski, M., et al., *J*. *Biol*. *Chem. ,* 271, 31543-31548 (1996)) and the following experiment was carried out in order to prove a hypothesis that, when yeast cells are made to coexist with FK 506 and rhodamine 6G, discharge of rhodamine 6G outside the cell is inhibited by a PDR5 transporter whereby rhodamine 6G is accumulated in the cell. Thus, the above yeast strain, KHW3 + pYI-PDR5 and KHW3 + pYI were subjected to a static culture in a microtiter plate at 32°C for 24 hours on an SD-uracil medium containing various concentrations of rhodamine 6G, absorbance at 540 nm was measured using a microplate reader and degree of growth was investigated. Concentration of rhodamine when degree of growth is 50% when no rhodamine 6G is added was defined as IC₅₀. As shown in Fig. 3, KHW3 + pYI-PDR5 showed resistance to rhodamine 6G having cytotoxicity and the IC₅₀ was 9.5 µg/ml while KHW3 + pYI showed a significantly high sensitivity to rhodamine 6G and IC₅₀ was 0.05 µg/ml. The result shows that, in the above screening method, rhodamine 6G is discharged by a PDR5 transporter.

Effect of FK 506 on intracellular accumulation of rhodamine 6G was investigated by measuring the rhodamine 6G concentration in the cell using a fluorometer. Wild strain (W303-1A + pYI) containing KHW3 + pYI-PDR5, KHW3 + pYI and pYI was incubated in an SD-uracil medium and kept with 25 µg/ml of FK 506 in a phosphate buffer (PBS) at 32°C for 2 hours. After keeping at 32°C for 30 minutes with 1 µg/ml of rhodamine 6G, washing with PBS was conducted and concentration of rhodamine 6G in the cell was measured by excitation light of 529 nm and fluorescence of 553 nm using a fluorometer of type F-2000 (Hitachi).

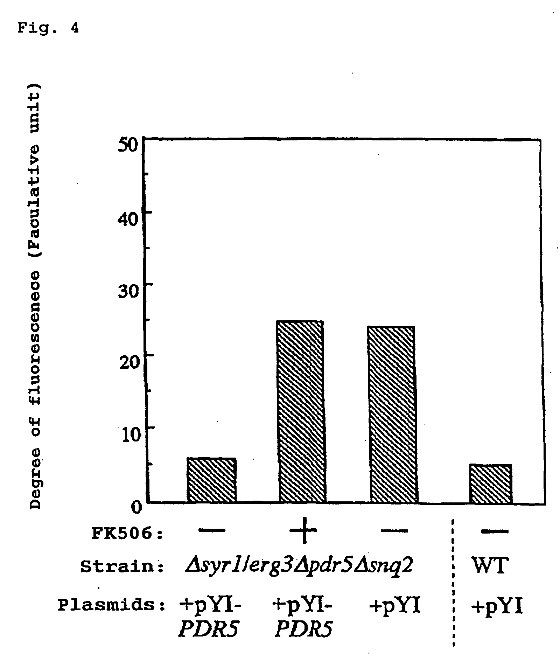

As shown in Fig. 4, KHW3 + pYI accumulated rhodamine 6G in the similar level to KHW3 + pYI-PDR5 in the presence of FK 506 while the accumulated amount of rhodamine 6G by KHW3 + pYI-PDR5 and W303-1A + pYI in the absence of FK 506 was lower than the accumulated amount by KHW3 + pYI-PDR5 in the presence of FK 506.

The above result shows that FK 506 is an inhibiting substance which is specific to a PDR5 transporter and also shows that the above screening method for a PDR5 transporter-inhibiting substance is effective. Further, when an SNQ2 transporter-inhibiting activity of FK 506 by the same agar well diffusion method was investigated where PDR5 was substituted with SNQ2 which is another ABC transporter, no inhibiting activity to SNQ2 transporter by FK 506 was noted. Accordingly, it has been confirmed that the ABC transporter-inhibiting activity of FK 506 is specific to a PDR5 transporter and that an inhibiting substance specific to a PDR5 transporter having no cytotoxicity is able to be efficiently screened.

### [Example 3] Purification and physico-chemical properties of KPI which is an inhibiting substance for a PDR5 transporter

*Actinomyces* (less than 10,000 strains) was incubated in a glucose-bouillon medium (1% glucose, 1% meat extract, 1% polypeptone and 0.3% NaCl; pH 7.0) and the culture supernatant liquid was subjected to a screening for a PDR5 transporter-inhibiting substance having low cytotoxicity by an agar well diffusion method mentioned in Example 1. The culture supernatant liquid (50 µl) was added to a well having a diameter of 8 mm and the plate was incubated at 32°C for 2 days. When an indicator bacterium was KHW3 + pYI-PDR5 in an agar medium containing cycloheximide or cerulenin, inhibition rings due to inhibition of growth were noted while, when the indicator bacterium was KHW3 + pYI in a medium to which no drug was added, a plurality of culture supernatant liquids having very small size of inhibition ring were noted. The PDR5 transporter was purified from a culture supernatant liquid of *Actinomyces* E-420 strain having a strong inhibiting activity to a PDR5 transporter among them. Since it was found that the *Actinomyces* E-420 strain belonged to genus *Kitasatospora* from bacteriological properties, sequence analysis, etc. of 16SrRNA, etc., the present microorganism was named *Kitasatospora* sp. Further, a PDR5 transporter-inhibiting substance produced by *Actinomyces* E-420 strain was named KPI (*Kitasatospora* PDR5 inhibitor). As mentioned in Example 1, one inhibition unit (IU) was calculated on the definition that a growth inhibiting activity showing an inhibition ring of 20 mm was defined as 20 inhibition units per ml (IU/ml).

KPI was purified from 4 L of culture supernatant liquid obtained by incubation of F-420 strain on a glucose-bouillon medium. The culture supernatant liquid (4 L) was concentrated by an evaporator to an extent of 5-fold and extracted with 1-butanol and the residue after evaporation in vacuo was dissolved in a solvent A (chloroform : methanol : water = 60:30:8, v/v/v). The dissolved substance was added to a Q-Sepharose column (manufactured by Pharmacia, Sweden) and the column was washed with the solvent A and methanol and eluted with a linear gradient concentration of the solvent A and a solvent B (chloroform : methanol : 4M sodium acetate = 60:30:8, v/v/v). An active fraction was collected, extracted with 1-butanol, concentrated and added to a column of Iatrobeads 6RS-8060 (1.5 × 80 cm; manufactured by Iatron) which was equilibrated with a solvent C (chloroform : methanol : water = 70:30:2, v/v/v). An active fraction was eluted with a linear gradient concentration of the solvent C and a solvent D (chloroform : methanol : water = 30:70:4, v/v/v). A PDR5 transporter-inhibiting substance KPI was further purified by a Sephadex LH-20 column chromatography and, from 4 L of a culture supernatant liquid of *Actinomyces* E-420 strain, about 4 mg of pure KPI were obtained in a yield of 7%.

Physico-chemical properties of KPI are shown in Table 2. KPI was a light yellow non-crystalline compound, showed no clear melting point and was decomposed at not lower than 180°C. Molecular weight of KPI according to an FAB-mass analysis was 1,193 and a fragment peak was noted at m/z 1042.42 by an FAB-MS-MS analysis. Although KIP was soluble in pyridine and dimethyl sulfoxide, it was not soluble in water, ethyl acetate and chloroform. KIP was stable between pH 7.0 and pH 9.0 and, even after heated at 65°C for 25 minutes, not less than 90% of PDR5 transporter-inhibiting activity remained. A methanolic solution of KPI had maximum absorptions at ultraviolet regions of 258 nm, 270 nm and 280 nm and was presumed to have a conjugated triene structure. The conjugated triene structure was stable even in an acidic condition.

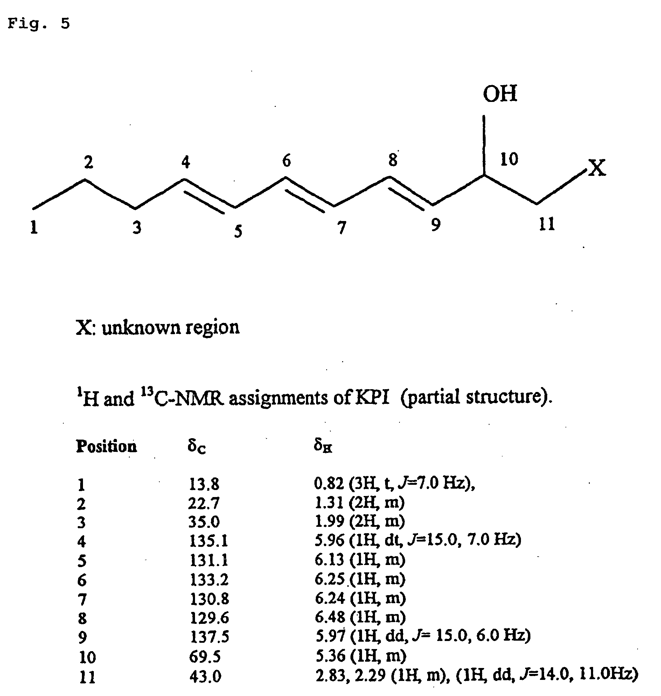

Analysis of KPI was carried out by means of COSY, HMBC, ROESY, TOCSY and HSQC. From the result of a ¹³C-NMR analysis in pyridine-d₅, 50 peaks were confirmed although precise numbers of carbon were unable to be determined. Twenty carbon atoms to which oxygen atom was bonded were found (99.9, 81.2, 80.4, 78.9. 77.7, 76.9, 75.8, 74,9, 74.7, 73.8, 73.2, 73.0, 72.8, 71.8, 71.7, 71.2, 70.9, .70.8, 69.5 and 69.0 ppm) and one of them (99.9 ppm) was found to be an acetal carbon. Fig. 5 shows a partial structure of KPI which was clarified by means of a ¹H-NMR analysis.

**Table 2**

| | KPI |
|---|---|
| Appearance | Light yellow powder |
| Melting point (°C) | 180-185 |
| Molecular weight | 1193; FAB-MS, 1,192 (M-H)⁺ |
| mUVλₘₐₓ (methanol) (nm) | 258, 270, 280 |
| Eluting time by HLPC (min) | 21.1 |
| pH Stability | 7-9; decomposed by 0.1% CF₃COOH |
| Solubility | Soluble in pyridine and dimethyl sulfoxide; insoluble in water, ethyl acetate and chloroform |

### [Example 4] Biological properties of KPI

Inhibition activity of KPI for a PDR5 transporter was analyzed in detail by an agar well diffusion method mentioned in Example 1. A triple gene disruption strain KHW3 containing pYI vector (KHW3 + pYI) and KHW3 containing a plasmid pYI-PDR5 highly expressing PDR5 (KHW3 + pYI-PDR5) were used as indication bacteria and the size of inhibition ring by growth inhibition in the co-presence of cycloheximide and cerulenin was compared with the size of the inhibition ring in the absence of both drugs and the inhibition activity of KPI for a PDR5 transporter was evaluated. As mentioned in Example 1, one inhibition unit (IU) was calculated on the basis of the definition that a growth inhibition activity showing an inhibition ring of 20 mm was defined as 20 inhibition units per ml (IU/ml).

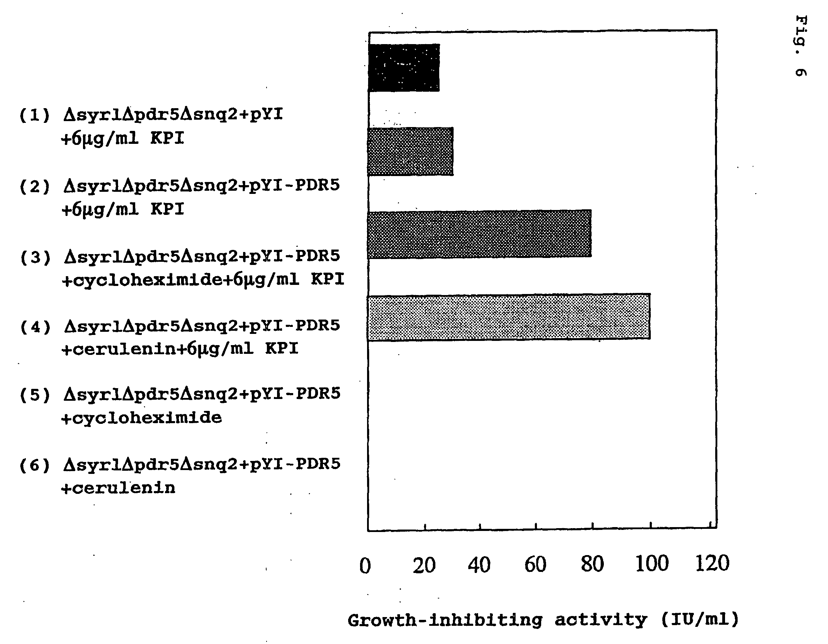

As shown in Fig. 6, KPI showed a transporter-inhibiting activity of 25 IU/ml and 30 IU ml/ml to KHW3 + pYI and KHW3 + pYI-PDR5, respectively by an agar well diffusion method mentioned in Example 1 and, if cycloheximide and cerulenin were made to co-exist, growth inhibition activity for KHW3 + pYI-PDR5 increased to an extent of 4-fold and 3-fold, respectively. Such a result shows that KPI inhibited the function of a PDR5 transporter or discharge of the drug outside the cells and, as a result, cycloheximide or cerulenin was accumulated in the yeast cell whereupon the yeast died. Further, because of the fact that SNQ2 which is a homolog of PDR5 and discharges 4-nitroquinoline oxide outside the cells has no inhibiting effect for an SNQ2 transporter of KIP, it was noted that KPI specifically inhibited a PDR5 transporter.

In order to confirm that KPI inhibits the drug-discharging function of a PDR5 transporter, the effect of KPI on discharge of a fluorescent dye rhodamine 123 outside the cells was tested by a method mentioned in Example 2. The effect of KPI on accumulation of rhodamine 123 in the cells was tested by measuring the concentration of rhodamine in the cell using a fluorometer. KHW3 + pYI-PDR5, KHW3 + pYI and a wild strain containing pYI (W303-1A + pYI) were incubated in an SD-uracil medium and kept in a phosphate buffer (PBS) with 0, 20 and 30 µg/ml of KPI at 32°C for 2 hours. After that, they were kept at 32°C for 30 minutes with 1 µg/ml of rhodamine 123 and washed with PBS and concentration of rhodamine in the cell was measured by exciting light of 505 nm and fluorescence of 534 nm using a fluorometer of type F-2000 (Hitachi).

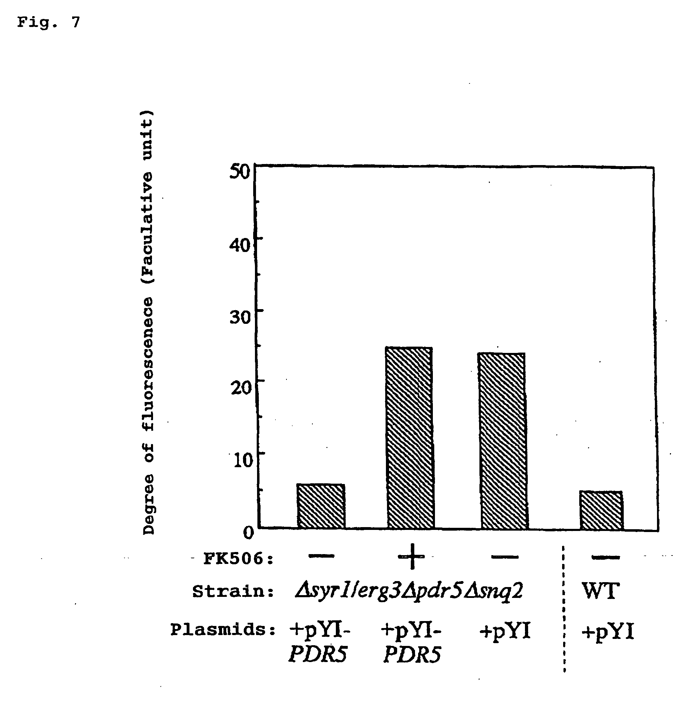

As shown in Fig. 7, accumulated amount of rhodamine 123 by W303-1A + pYI in the absence of KPI and that by KHW3 + pYI-PDR5 in the presence of KPI were low while accumulated amount of rhodamine 123 by KHW3 + pYI-PDR5 in the presence of 20 and 30 µg/ml of KPI increased depending upon the concentration of KPI and reached 3-fold and 4-fold, respectively of the accumulated amount of KHW3 + pYI-PDR5 in the absence of KPI being in the same level as the accumulated amount of KHW3 + pYI in the absence of KPI.

The above result shows that KPI is a specific inhibitor for a PDR5 transporter and also that a screening method for a PDR5 transporter-inhibitor mentioned in Example 1 is effective. When PDR5 was substituted with SNQ2 which was another ABC transporter and an SNQ2 transporter-inhibiting activity of KPI was tested by the same agar well diffusion method, no SNQ2 transporter-inhibiting activity was noted in KPI. Accordingly, it was confirmed that an ABC transporter-inhibiting activity of KPI was specific to a PDR5 transporter and that a specific inhibiting substance for a PDR5 transporter having a low cytotoxicity was able to be efficiently screened by the above agar well diffusion method.

### Industrial Applicability

In the present invention, yeast cells having a high drug sensitivity are used as a host whereby inhibiting activity or promoting activity of a test substance to active transport of ABC protein which is a genetic product of ABC protein gene introduced into the host can be efficiently evaluated. Thus, ABC protein gene is introduced into yeast cells having a high drug sensitivity to prepare transformed yeast cells in which ABC protein is expressed in the cell membrane site and, when such cells are used, it is now possible to efficiently and easily screen a promoting substance or an inhibiting substance for ABC protein from many test substances such as culture supernatant liquid of microorganisms.

The present invention also provides a promoting substance or an inhibiting substance for ABC protein prepared by the above screening method.

A promoting substance for ABC protein or a pharmaceutical composition containing the same is useful as a drug which maintains or enhances the biodefense system. On the other hand, an inhibiting substance for ABC protein or a pharmaceutical composition containing the same is useful as a drug for the prevention of acquisition of multi-drug resistance or for suppression thereof.

## Claims

1. A method for screening a substance which promotes or inhibits an active transfer of ABC protein, **characterized in that**, (a) ABC protein gene is introduced into a yeast cell having a high drug sensitivity to prepare a transformed yeast cell where ABC protein is expressed in cell membrane site, (b) the transformed yeast cell is incubated in the presence of a test substance and a substance which is subjected to an active transfer outside the cell by ABC protein existing in the cell membrane site and has a cytotoxicity to the yeast cell and (c) degree of the growth of said transformed yeast cell is compared with the case where incubation is carried out in the absence of the test substance.

2. A method for screening a substance having a low cytotoxicity which promotes or inhibits an active transfer of ABC protein, **characterized in that**, the substance which promotes or inhibits the active transfer of ABC protein screened by the method mentioned in claim 1 is subjected to measurement of a growth inhibiting action to a yeast cell having a high drug sensitivity where ABC protein is not expressed.

3. The method for screening according to any of claims 1 and 2, wherein the ABC protein gene is a gene participating in acquisition of multi-drug resistance.

4. The method for screening according to claim 3, wherein the gene participating in acquisition of multi-drug resistance is PDR5 gene of yeast.

5. The method for screening according to any of claims 1 to 4, wherein the yeast cell having a high drug sensitivity is a variant yeast cell where permeation of drug through cell membrane rises than a wild strain.

6. The method for screening according to claim 5, wherein the variant yeast cell is a variant having variation to cell membrane lipid synthetic gene.

7. The method for screening according to claim 6, wherein the variant having variation to the cell membrane lipid synthesis gene is a deficient variant of SYR1/ERG3 gene or gene disruption strain.

8. A transformed yeast cell where ABC protein is expressed in a cell membrane site by introduction of ABC protein gene into a yeast cell having a high drug sensitivity.

9. A compound which is obtainable by the method for screening mentioned in any of claims 1 to 7.

10. A compound represented by a partial structure represented by the following formula (1): 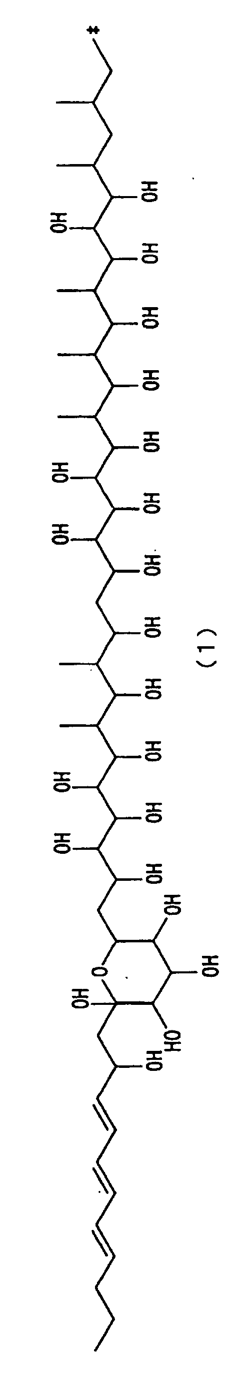 , having a molecular weight of 1193.7 and having an action of inhibiting a material transport owned by ABC protein or a reduced substance, an ether substance or an ester substance thereof.

11. The compound according to claim 10 which is obtained from a culture supernatant liquid of *Actinomyces* E-420 strain or a reduced substance, an ether substance or an ester substance thereof.

12. An ABC protein-inhibitor which is **characterized in** containing a compound represented by the formula (2):
A-B-C (2)
{in the formula,
A is a formula (3): 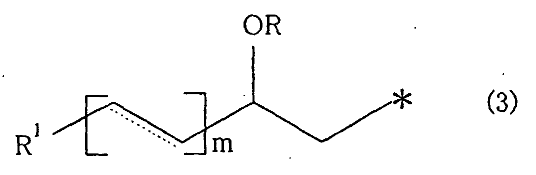 (in the formula, R¹ is hydrogen or a C₁₋₆ lower alkyl; R is hydrogen or a substituent for hydroxy group; m is 3, 4 or 5; and the group in brackets is -CH₂-CH₂- or -CH=CH-.);
B is a formula (4-1): 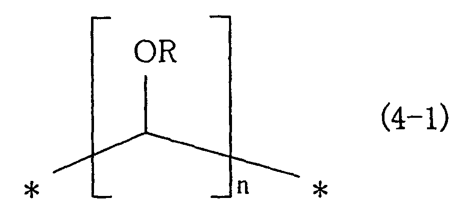 (in the formula, R may be same or different for each constituting unit and is the same as that defined above; and n is 4, 5 or 6) or a formula (4-2): 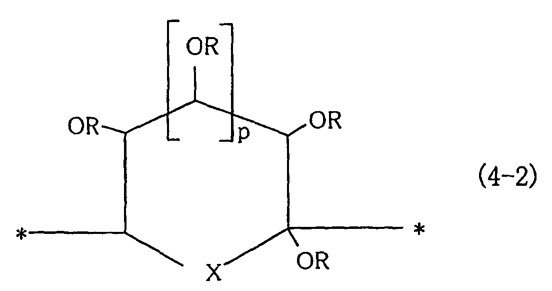 (in the formula, R may be same or different for each constituting unit and is the same as that defined above; p is 0, 1 or 2; and X is O, S or NH);
C is a formula (5):
D-E-F-G-Z (5)
(in the formula, D is the formula (6): 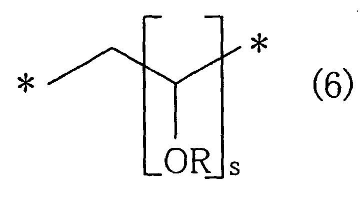 (in the formula, R may be same or different for each constituting unit and is the same as that defined above; and s is 4, 5 or 6);
E is a formula (7): 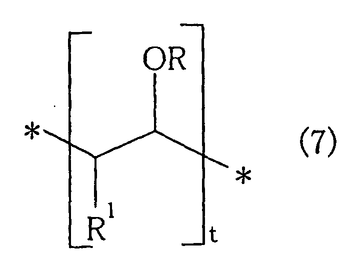 (in the formula, R and R¹ may be same or different for each constituting unit and is the same as that defined above; and t is 2, 3 or 4);
F is a formula (8): 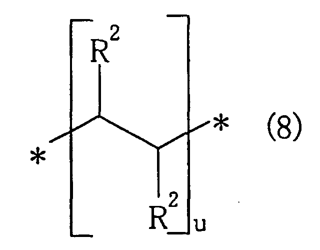 (in the formula, R² may be same or different for each constituting unit or may be same or different in the constituting unit and is OR (R is the same as that defined above), hydrogen or a C₁₋₆ lower alkyl group; and u is 1 or 2);
G is a formula (9): 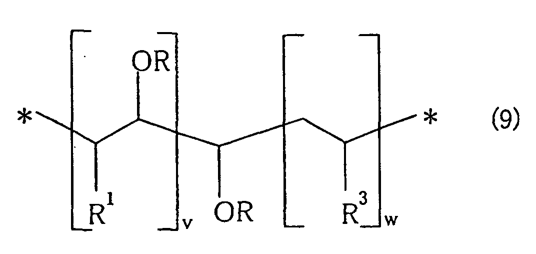 (in the formula, R may be same or different for each constituting unit and is the same as that defined above; R¹ may be same or different for each constituting unit and is the same as that defined above; R³ may be same or different for each constituting unit and is a C₁₋₆ lower alkyl group; v is 3, 4 or 5; and w is 1, 2 or 3); and
Z is a substituent).

13. The ABC protein-inhibitor according to claim 12, wherein, in the formula (2) mentioned in claim 12, the group represented by the symbol A is the following formula (10): 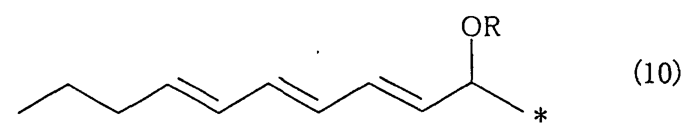 (in the formula, R is hydrogen or a substituent for a hydroxy group).

14. The ABC protein-inhibitor according to claim 12 or 13, wherein, in the formula (2) mentioned in claim 12, the group represented by the symbol B is the following formula (11): 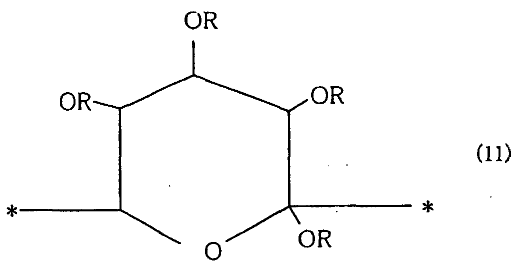 (in the formula, R may be same or different for each constituting unit and is hydrogen or a substituent for a hydroxy group).

15. The ABC protein-inhibitor according to any of claims 12 to 14, wherein, in the formula (2) mentioned in claims 12, s is 5, t is 3, u is 1 or 2, v is 4 and w is 2.

16. An ABC protein-inhibitor which is **characterized in** containing a compound represented by the formula (12): 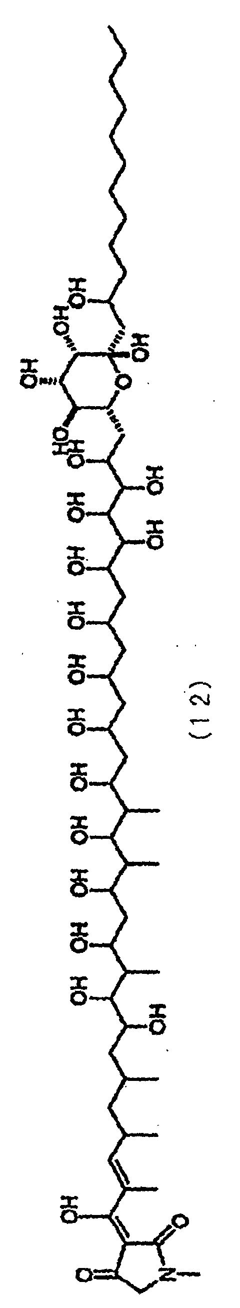 or an ether substance or an ester substance thereof or a compound represented by the formula (13): 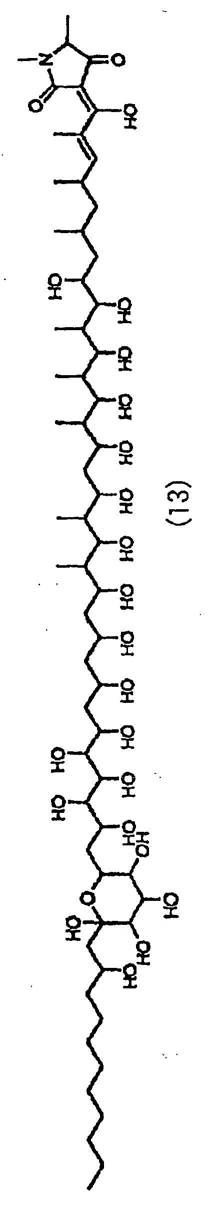 or an ether substance or an ester substance thereof.

17. The ABC protein-inhibitor according to any of claims 12 to 16, wherein the ABC protein is an ABC protein participating in acquisition of multi-drug resistance.

18. The ABC protein-inhibitor according to claim 17, wherein the ABC protein is PDR5 of yeast.
